# EUROPÄISCHE PATENTSCHRIFT

(11) **EP 3 652 138 B1**
(45) Veröffentlichungstag und Bekanntmachung des Hinweises auf die Patenterteilung: **30.06.2021**
(21) Anmeldenummer: 18737312.1
(22) Anmeldetag: 12.07.2018
(51) Int. Cl.: C07C 5/333, C07C 7/00, C07C 7/04, C07C 7/09, C07C 9/08, C07C 11/06, C10G 9/00, C10G 9/36, C10G 51/06

(54) **PROZESS UND ANLAGE ZUR HERSTELLUNG VON PROPYLEN DURCH KOMBINATION VON PROPANDEHYDRIERUNG UND DAMPFSPALTVERFAHREN MIT VORTRENNSCHRITTEN IN BEIDEN VERFAHREN ZUR TEILWEISE ENTFERNUNG VON WASSERSTOFF UND METHAN**
PROCESS AND PLANT FOR PRODUCING PROPYLENE BY COMBINATION OF PROPANE DEHYDRATION AND STEAM CRACKING WITH PRE-SEPARATION STEPS IN BOTH PROCESSES FOR REMOVING HYDROGEN AND METHANE
PROCÉDÉ ET INSTALLATION DE FABRICATION DE PROPYLÈNE PAR COMBINAISON DE DÉSHYDROGÉNATION DE PROPANE ET PROCÉDÉ DE VAPOCRAQUAGE AVEC STAGES DE PRE-SÉPARATION DANS LES DEUX PROCÉDÉS POUR ÉLIMINER HYDROGÈNE ET MÉTHANE

(30) Priorität: 12.07.2017 EP 17180974
(43) Veröffentlichungstag der Anmeldung: 20.05.2020
(73) Patentinhaber: Linde GmbH, 82049 Pullach (DE)
(72) Erfinder: HÖFEL, Dr. Torben, 81543 München (DE); TÖGEL, Christine, 80331 München (DE); ZELLHUBER, Dr. Mathieu, 82152 Planegg (DE); LAIB, Heinrich, 67056 Ludwigshafen (DE); KOTREL, Stefan, 67056 Ludwigshafen (DE); DIETERLE, Martin, 67056 Ludwigshafen (DE); PATCAS, Florina Corina, 67056 Ludwigshafen (DE); GIESA, Sonja, 67056 Ludwigshafen (DE)
(86) Internationale Anmeldenummer: PCT/EP2018/068996
(87) Internationale Veröffentlichungsnummer: WO 2019/012062

(56) Entgegenhaltungen:
- CN-A- 103 086 826
- DE-A1-102005 053 732

## Beschreibung

Die Erfindung betrifft einen Prozess und eine Anlage zur Herstellung von Propylen sowie ein Verfahren zum Umrüsten einer Anlage zum Dampfspalten gemäß den Oberbegriffen der unabhängigen Patentansprüche.

### Stand der Technik

Propylen (Propen) wird herkömmlicherweise überwiegend durch Dampfspalten (engl. Steam Cracking) von Kohlenwasserstoffeinsätzen und weitere Umwandlungsverfahren im Zuge von Raffinerieprozessen hergestellt. In diesen Fällen stellt Propylen ein in geringerem Umfang anfallendes Nebenprodukt dar. Aufgrund des zunehmenden Bedarfs an Propylen, insbesondere für Polypropylen, wird daher auch die Propandehydrierung eingesetzt.

Die Propandehydrierung ist ein allgemein bekanntes Verfahren der petrochemischen Industrie und beispielsweise im Artikel "Propene" in Ullmann's Encyclopedia of Industrial Chemistry, Onlineausgabe 16. September 2013, DOI: 10.1002/14356007.a22_211.pub3, insbesondere Abschnitt 3.3.1, "Propane Dehydrogenation" beschrieben.

Bei der Propandehydrierung handelt es sich um eine endotherme Gleichgewichtsreaktion, die im Allgemeinen an Edel- oder Schwermetallkatalysatoren, umfassend beispielsweise Platin oder Chrom, durchgeführt wird. Die Dehydrierungsreaktion ist ausgesprochen selektiv. Für kommerziell verfügbare Prozesse werden Gesamtausbeuten von ca. 90% genannt. Ungeachtet dieser hohen Selektivität entstehen neben dem abgespaltenen Wasserstoff jedoch typischerweise auch geringere Mengen an Kohlenwasserstoffen mit einem, zwei sowie vier und mehr als vier Kohlenstoffatomen als Nebenprodukte. Diese Nebenprodukte müssen vom Zielprodukt Propylen abgetrennt werden.

Dampfspaltverfahren und Raffinerieprozesse, in denen Propylen gebildet wird, sind ebenfalls in der Literatur beschrieben, beispielsweise im Artikel "Ethylene" in Ullmann's Encyclopedia of Industrial Chemistry, Onlineveröffentlichung 15. April 2009, DOI: 10.1002/14356007.a10_045.pub3, und im Artikel "Oil Refining" in Ullmann's Encyclopedia of Industrial Chemistry, Onlineveröffentlichung 15. Januar 2007, DOI: 10.1002/14356007.a18_051 .pub2.

Grundsätzlich kann eine Aufreinigung eines bei der Propandehydrierung erzeugten Komponentengemischs zumindest teilweise zusammen mit der Aufreinigung eines Propylen enthaltenden Komponentengemischs aus einem anderen Verfahren, in dem Propylen gebildet wird, beispielsweise einem Dampfspaltverfahren oder einem Raffinerieprozess, erfolgen.

Die Kombination der Aufreinigung eines Komponentengemischs aus einer Propandehydrierung mit der Aufreinigung eines Komponentengemisch aus einem Dampfspaltverfahren ist beispielsweise aus der US 4,458,096 A oder, spezifisch bezogen auf eine Spaltung von Kohlenwasserstoffen mit zwei Kohlenstoffatomen, aus der WO 2015/128039 A1 bekannt, eine entsprechende Kombination mit der Aufreinigung eines Komponentengemisch aus einem Fluid-Catalytic-Cracking-Verfahren beispielsweise aus der US 8,563,793 A oder der US 2010/331589 A1. Genauere Ausführungen bezüglich einer entsprechenden Kombination enthalten diese Druckschriften jedoch nicht. Ferner sei bereits an dieser Stelle darauf hingewiesen, dass ein Fluid-Catalytic-Cracking-Verfahren Produktgemische mit grundsätzlich anderer Zusammensetzung als ein Dampfspaltverfahren liefert, so dass eine kombinierte Trennung hier abweichend ausgestaltet werden muss.

Die vorliegende Erfindung stellt sich die Aufgabe, Prozesse zur Herstellung von Propylen, bei denen ein Komponentengemisch aus einem Verfahren zur Propandehydrierung und ein Komponentengemisch aus einem Dampfspaltverfahren gemeinsam aufgereinigt werden, zu verbessern und effizienter zu gestalten.

### Offenbarung der Erfindung

Vor diesem Hintergrund schlägt die vorliegende Erfindung einen Prozess und eine Anlage zur Herstellung von Propylen sowie ein Verfahren zum Umrüsten einer Anlage zur Durchführung eines Dampfspaltverfahrens mit den jeweiligen Merkmalen der unabhängigen Patentansprüche vor. Bevorzugte Ausgestaltungen sind Gegenstand der abhängigen Patentansprüche sowie der nachfolgenden Beschreibung.

Für eine kombinierte Aufreinigung von Komponenten aus unterschiedlichen Verfahren ist es besonders vorteilhaft, wenn die jeweiligen Komponentengemische gleiche bzw. ähnliche Komponenten enthalten, d.h. die Komponentengemische sich nicht gegenseitig mit bestimmten, in dem jeweils anderen Komponentengemisch nicht enthaltenen Komponenten "kontaminieren" (beispielsweise mit Wasserstoff, Kohlendioxid oder Oxygenaten).

Eine Kombination der Aufreinigung ist insbesondere dann vorteilhaft, wenn die jeweiligen Komponentengemische einen ähnlichen Konzentrationsbereich aufweisen, so dass ein synergetischer Trennprozess erwartet werden kann. Dies ist jedoch in der Praxis normalerweise nicht der Fall. Ferner erfolgt eine Kombination der Aufreinigung vorteilhafterweise dann, wenn eines der Verfahren deutlich geringere Mengen eines entsprechenden Komponentengemischs liefert bzw. eine entsprechende Anlage kleiner ausgebildet und daher eine separate Aufreinigung nicht lohnenswert ist. Dies kann insbesondere der Fall sein, wenn das Dampfspaltverfahren bereits in Form einer Anlage realisiert ist und eine Propandehydrierung mit deutlich geringer Kapazität nachgerüstet wird. Dies könnte insbesondere vorteilhaft sein, wenn einige Anlagenteile zur Durchführung des Dampfspaltverfahrens aufgrund einer späteren Einsatzänderung nicht mehr mit voller Kapazität laufen und diese Kapazitäten entsprechend von dem Propandehydrierungsverfahren genutzt werden können.

Ein wesentlicher Aspekt der vorliegenden Erfindung ist es, ein Komponentengemisch, das unter Verwendung eines Verfahrens zur Propandehydrierung erhalten wird, nachfolgend auch als "erstes" Komponentengemisch bezeichnet, derart vorzubehandeln, dass dieses in einem an (zumindest) Wasserstoff abgereicherten Zustand und insbesondere bei einem erhöhten Druck vorliegt. Hierbei wird das erste Komponentengemisch einem oder mehreren Vortrennschritten unterworfen, die nachfolgend auch als "erste" Vortrennschritte bezeichnet werden. Das auf diese Weise vorbehandelte Komponentengemisch, das nachfolgend, wenn konkret hierauf Bezug genommen wird, auch als "drittes" Komponentengemisch bezeichnet wird, enthält aufgrund seiner Vorbehandlung überwiegend Kohlenwasserstoffe mit drei Kohlenstoffatomen. Ferner können geringere Mengen an Methan, Restwasserstoff sowie Kohlenwasserstoffe mit zwei Kohlenstoffatomen sowie Kohlenwasserstoffe mit vier und gegebenenfalls mehr als vier Kohlenstoffatomen enthalten sein.

Ein weiterer wesentlicher Aspekt der vorliegenden Erfindung ist es ferner, ein Komponentengemisch, das unter Verwendung eines Dampfspaltverfahrens erhalten wird, nachfolgend auch als "zweites" Komponentengemisch bezeichnet, derart vorzubehandeln, dass dieses in einem (zumindest) an Wasserstoff und Methan abgereicherten Zustand und insbesondere ebenfalls bei einem erhöhten Druck vorliegt. Hierbei wird das zweite Komponentengemisch einem oder mehreren Vortrennschritten unterworfen, die nachfolgend auch als "zweite Vortrennschritte" bezeichnet werden. Das auf diese Weise vorbehandelte Komponentengemisch, das nachfolgend, wenn konkret hierauf Bezug genommen wird, auch als "viertes" Komponentengemisch bezeichnet wird, enthält aufgrund seiner Vorbehandlung vorteilhafterweise überwiegend ähnliche Kohlenwasserstoffe, wie sie in dem dritten Komponentengemisch enthalten sind, und diese in einem vergleichbaren Konzentrationsbereich, sowie vergleichbare Mengen an Restwasserstoff und Restmethan, soweit nicht vollständig abgetrennt. Das zweite und das vierte Komponentengemisch können aber auch signifikante Mengen an Kohlenwasserstoffen mit vier Kohlenstoffatomen enthalten. Beispielsweise ist dies der Fall, wenn das vierte Komponentengemisch im Sumpf einer Deethanisierungskolonne vorliegt, wie nachfolgend im Detail erläutert.

Da grundsätzlich bei einem Dampfspaltverfahren größere Mengen an Kohlenwasserstoffen mit zwei Kohlenstoffatomen, insbesondere Ethan und Ethylen, gebildet werden können, insbesondere bei Einsatz von leichteren Dampfspalteinsätzen, kann im Zuge des oder der zweiten Vortrennschritte auch eine Abreicherung an Kohlenwasserstoffen mit zwei Kohlenstoffatomen im Zuge der Abreicherung an Wasserstoff und Methan erfolgen. Mit anderen Worten kann im Zuge des oder der zweiten Vortrennschritte ein Demethanizer-First-Verfahren oder ein Deethanizer-First-Verfahren zum Einsatz kommen. Auch der Einsatz eines Depropanizer-First-Verfahrens ist grundsätzlich möglich. Weitere Details werden nachfolgend erläutert. Auch im Zuge des oder der ersten Vortrennschritte kann jedoch bereits eine Abreicherung an Kohlenwasserstoffen mit zwei Kohlenstoffatomen erfolgen, falls dies zweckmäßig ist.

Die durch den oder die ersten Vortrennschritte und durch den oder die zweiten Vortrennschritte in ihrer Zusammensetzung und ihrem Druck zumindest teilweise aneinander angeglichenen dritten und vierten Komponentengemische können in besonders vorteilhafter Weise vereinigt und anschließend nachfolgenden, gemeinsamen Trennschritten unterworfen werden. Dies ermöglicht es, entsprechende Anlagenteile für beide Verfahren gemeinsam auszubilden und damit eine entsprechende Anlage mit geringeren Investitionskosten zu erstellen und/oder mit geringeren Betriebskosten zu betreiben.

Ist hier davon die Rede, dass ein Komponentengemisch gegenüber einem anderen Komponentengemisch, hier insbesondere das dritte gegenüber dem ersten und das vierte gegenüber dem zweiten, an einer oder an mehreren Komponenten, hier insbesondere an Wasserstoff bzw. Wasserstoff und Methan, "abgereichert" ist, sei hierunter verstanden, dass das abgereicherte Komponentengemisch höchstens den 0,5-fachen, 0,2-fachen, 0,1-fachen, 0,01-fachen oder 0,001-fachen Gehalt an der einen oder den mehreren Komponenten, bezogen auf das nicht abgereicherte Komponentengemisch und auf molarer, Masse- oder Volumenbasis, enthält. Auch eine vollständige Entfernung, d.h. eine "Abreicherung auf null" wird hier als Abreicherung verstanden. Nachfolgend bezeichnet der Begriff "überwiegend" einem Gehalt von wenigstens 60%, 80%, 90%, 95% oder 99% auf molarer, Masse- oder Volumenbasis.

Insgesamt schlägt die vorliegende Erfindung dabei einen Prozess zur Herstellung von Propylen vor, der das Durchführen eines Verfahrens zur Propandehydrierung unter Erhalt eines ersten Komponentengemischs, das zumindest Wasserstoff, Ethan, Ethylen, Propan und Propylen enthält, und das Durchführen eines Dampfspaltverfahrens unter Erhalt eines zweiten Komponentengemischs, das zumindest Wasserstoff, Methan, Ethan, Ethylen, Propan und Propylen enthält, vor. Zu den Details der jeweiligen Verfahren und den dabei typischerweise gebildeten Produktzusammensetzungen, insbesondere auch bezüglich zusätzlich zu den erwähnten Komponenten enthaltener Verbindungen, sei auf die mehrfach zitierte Fachliteratur verwiesen. Dem Verfahren zur Propandehydrierung werden dabei vorteilhafterweise Propan enthaltende Einsätze, dem Dampfspaltverfahren kohlenwasserstoffreiche Einsätze zugeführt. Bei letzteren handelt es sich beispielsweise um Naphtha, aber gegebenenfalls auch leichtere oder schwerere Einsätze, also solche, die leichter und/oder schwerer siedende Kohlenwasserstoffe enthalten, als sie typischerweise in Naphtha vorhanden sind.

Wie insoweit auch in kombinierten Prozessen üblich, erfolgt dabei ein Bilden eines ersten Trennungsprodukts, das zumindest überwiegend Propylen enthält, unter Verwendung zumindest eines Teils des Propylens des ersten und des zweiten Komponentengemischs und unter Einsatz eines oder mehrerer erster Trennschritte und das Bilden eines zweiten Trennungsprodukts, das zumindest überwiegend Propan enthält, unter Verwendung zumindest eines Teils des Propans des ersten und des zweiten Komponentengemischs und unter Einsatz des oder der ersten Trennschritte. Der oder die ersten Trennschritte können dabei insbesondere die Verwendung eines sogenannten C3-Splitters umfassen, dem kopfseitig das erste Trennungsprodukt und dem sumpfseitig das zweite Trennungsprodukt entnommen werden kann. Einem derartigen C3-Splitter sind typischerweise weitere Trennschritte vorgeschaltet, wie sie auch nachfolgend noch erläutert werden.

Insbesondere kann der Einsatz eines entsprechenden C3-Splitters typischerweise einem sogenannten Depropanizer, bzw. einer entsprechenden Depropanizerkolonne, entnommen werden wie grundsätzlich ebenfalls aus der zitierten Fachliteratur bekannt. Bei einem Depropanizer handelt es sich um eine Rektifikationskolonne, der kopfseitig eine gasförmige Fraktion, die überwiegend oder ausschließlich Kohlenwasserstoffe mit drei Kohlenstoffatomen enthält, entnommen werden kann, und der sumpfseitig eine flüssige Fraktion, die überwiegend oder ausschließlich Kohlenwasserstoffe mit vier und gegebenenfalls mehr Kohlenstoffatomen enthält, entnommen werden kann. Auch weiteren, einer entsprechenden Rektifikationskolonne zugeordneten Apparaten, die Teil eines entsprechenden Depropanizers sind, beispielsweise Absorbern, können anstelle der Rektifikationskolonne selbst oder zusätzlich zu dieser, entsprechende Fraktionen entnommen werden. Die kopfseitig dem Depropanizer oder entsprechenden Apparaten entnommene Fraktion kann dem C3-Splitter zugeführt werden. Ein entsprechender Depropanizer kann an unterschiedlicher Stelle in einer Trennsequenz zur Bearbeitung eines Komponentengemischs, insbesondere eines durch ein Dampfspaltverfahren erhaltenen Komponentengemischs, angeordnet sein. Insbesondere kann ein entsprechender Depropanizer im Rahmen der vorliegenden Erfindung stromab eines Deethanizers bzw. einer entsprechenden Deethanizerkolonne angeordnet und zur trenntechnischen Bearbeitung eines Sumpfprodukts des Deethanizers eingerichtet sein. Wie auch nachfolgend erläutert, kann im Rahmen der vorliegenden Erfindung ein Deethanizer im Zuge des oder der zweiten Vortrennschritte oder bereits im Zuge des oder der ersten Trennschritte eingesetzt werden, abhängig davon, ob ein Deethanizer-First-Verfahren oder ein Demethanizer-First-Verfahren zum Einsatz kommt. Entsprechendes gilt auch für einen Depropanizer, der ebenfalls an erster Stelle einer entsprechenden Trennsequenz stehen kann.

Die vorliegende Erfindung umfasst ferner das Bilden eines dritten Trennungsprodukts, das zumindest überwiegend Ethylen enthält, unter Verwendung zumindest eines Teils des Ethylens des ersten und des zweiten Komponentengemischs und unter Einsatz eines oder mehrerer zweiter Trennschritte, sowie das Bilden eines vierten Trennungsprodukts, das zumindest überwiegend Ethan enthält, unter Verwendung zumindest eines Teils des Ethans des ersten und des zweiten Komponentengemischs und unter Einsatz des oder der zweiten Trennschritte. Der oder die zweiten Trennschritte umfassen dabei typischerweise die Verwendung eines sogenannten C2-Splitters, dem kopfseitig das dritte Trennungsprodukt und sumpfseitig das vierte Trennungsprodukt entnommen werden kann. Ein entsprechender C2-Splitter kann insbesondere mit einer überwiegend oder ausschließlich Ethan und Ethylen enthaltenen Fraktion gespeist werden, die in einem Demethanizer-First-Verfahren vom Kopf eines Deethanizers, also einer entsprechenden Rektifikationskolonne oder einem dieser zugeordneten Apparat, und in einem Deethanizer-First-Verfahren aus dem Sumpf eines Demethanizers abgezogen werden kann. Sämtliche Verfahrensvarianten werden auch nachfolgend unter Bezugnahme auf die Zeichnungen noch näher erläutert und können im Rahmen der vorliegenden Erfindung zum Einsatz kommen.

Wie erwähnt, umfasst die vorliegende Erfindung Vortrennschritte, wobei vorgesehen ist, dass zumindest ein Teil des ersten Komponentengemischs unter Erhalt eines dritten Komponentengemischs einem oder mehreren ersten Vortrennschritten unterworfen wird, der oder die eine Druckerhöhung und eine zumindest teilweise Entfernung von Wasserstoff umfassen, und dass zumindest ein Teil des zweiten Komponentengemischs unter Erhalt eines vierten Komponentengemischs einem oder mehreren zweiten Vortrennschritten unterworfen wird, der oder die eine Druckerhöhung, eine zumindest teilweise Entfernung von Wasserstoff und eine zumindest teilweise Entfernung von Methan umfassen. Bereits an dieser Stelle sei darauf hingewiesen, dass, wie bereits oben erwähnt, im Rahmen des oder der zweiten Vortrennschritte auch eine zumindest teilweise Entfernung von Kohlenwasserstoffen mit zwei Kohlenstoffatomen vorgenommen werden kann. In letzterem Fall kommt die vorliegende Erfindung im Zusammenhang mit einem Deethanizer-First-Verfahren zum Einsatz, ansonsten im Zusammenhang mit einem Demethanizer-First-Verfahren. Wie erwähnt, kann die vorliegende Erfindung auch im Zusammenhang mit einem Depropanizer-First-Verfahren eingesetzt werden.

Ferner ist im Rahmen der vorliegenden Erfindung vorgesehen, dass zumindest ein Teil des dritten Komponentengemischs gemeinsam mit zumindest einem Teil des vierten Komponentengemischs dem oder den ersten Trennschritten unterworfen wird. Die vorliegende Erfindung sieht dabei in ihren nachfolgend erläuterten, jeweiligen Ausgestaltungen unterschiedliche Möglichkeiten zur Vereinigung des dritten Komponentengemischs mit dem vierten Komponentengemisch bzw. ihrer jeweils verwendeten Anteile vor. In sämtlichen Fällen besteht ein Hauptvorteil der vorliegenden Erfindung darin, dass aufgrund einer vergleichbaren Zusammensetzung des dritten und vierten Komponentengemischs in zumindest einigen der Komponenten eine besonders einfache und effiziente gemeinsame Trennung möglich ist.

Wie bereits erwähnt, kann die vorliegende Erfindung im Zusammenhang mit einem Deethanizer-First-Verfahren zum Einsatz kommen. In diesem Fall werden bei der Entfernung von Wasserstoff und Methan in dem oder den zweiten Vortrennschritten auch Ethan und Ethylen zumindest überwiegend entfernt, so dass diese überwiegend nicht aus dem zweiten in das vierte Komponentengemisch bzw. entsprechende Anteile übergehen. Es sei darauf hingewiesen, dass in einem entsprechenden Deethanizer-First-Verfahren das vierte Komponentengemisch auch ein Sumpfprodukt eines Deethanizers darstellen kann, das im Sumpf einer entsprechenden Rektifikationskolonne vorliegt.

Bei einer "Rektifikationskolonne" handelt es sich im hier verwendeten Sprachgebrauch um eine Trenneinheit, die dafür eingerichtet ist, ein oder mehrere gasförmig oder flüssig oder in Form eines Zweiphasengemischs mit flüssigen und gasförmigen Anteilen, ggf. auch im überkritischen Zustand, eingespeiste Komponentengemische durch Rektifikation zumindest teilweise aufzutrennen, also aus dem oder den Komponentengemischen jeweils Reinstoffe oder zumindest Stoffgemische mit anderer Zusammensetzung zu erzeugen. Die Rektifikation umfasst dabei bekanntermaßen wiederholte Verdampfungs- und Kondensationsvorgänge, insbesondere auf bzw. unter Verwendung von hierfür geeigneten Einbauten, beispielsweise Trennböden oder geordneten oder ungeordneten Packungen. Eine Rektifikationskolonne zum Einsatz im Rahmen der vorliegenden Erfindung weist einen Sumpfverdampfer auf. Hierbei handelt es sich um eine Einrichtung mit einem Wärmetauscher, der beheizt wird und dafür eingerichtet ist, eine im Sumpf der Rektifikationskolonne anfallende flüssige Fraktion, auch als Sumpfflüssigkeit bezeichnet, zu erwärmen. Mittels eines Sumpfverdampfers wird kontinuierlich ein Teil des Sumpfprodukts verdampft und gasförmig in die Rektifikationskolonne zurückgespeist. Eine Rektifikationskolonne zum Einsatz im Rahmen der vorliegenden Erfindung ferner einen Kopfkondensator auf, der in der Rektifikationskolonne aufsteigendes Gas kondensiert und in kondensiertem Zustand auf die Rektifikationskolonne zurückführt.

Zur Auslegung und spezifischen Ausgestaltung von Rektifikationskolonnen und weiteren Trenneinrichtungen sei auf einschlägige Lehrbücher verwiesen (siehe beispielsweise K. Sattler, "Thermische Trennverfahren: Grundlagen, Auslegung, Apparate", 3. Auflage, Wiley-VCH, Weinheim 2001).

Zu Trennverfahren, wie sie spezifisch zur Behandlung von Komponentengemischen eingesetzt werden, die unter Verwendung von Dampfspaltverfahren gebildet werden, insbesondere Trennverfahren, die eine Demethanisierung und eine Deethanisierung umfassen, sei auf den bereits zitierten Artikel "Ethylene" in Ullmann's Encyclopedia of Industrial Chemistry verwiesen. Derartige Trennverfahren unterscheiden sich insbesondere durch die Reihenfolge der jeweiligen Trennschritte. So sind beispielsweise die bereits mehrfach erwähnten Demethanizer-First-Verfahren (auch als Front-End-Demethanizer-Verfahren bezeichnet) und die ebenfalls mehrfach erwähnten Deethanizer-First-Verfahren (auch als Front-End-Deethanizer-Verfahren bezeichnet), aber auch Depropanizer-First-Verfahren (auch als Front-End-Depropanizer-Verfahren bezeichnet) bekannt. Wie auch nachfolgend noch im Detail erläutert, eignet sich die vorliegende Erfindung insbesondere zum Einsatz im Zusammenhang mit Deethanizer-First-Verfahren, aber auch zum Einsatz mit Demethanizer-First-Verfahren oder Depropanizer-First-Verfahren.

Insbesondere können Demethanizer, Deethanizer und Depropanizer, wie sie bereits zuvor erwähnt wurden, als entsprechende Rektifikationskolonnen ausgebildet sein bzw. derartige Rektifikationskolonnen umfassen, die nachfolgend auch als Demethanisierungskolonnen, Deethanisierungskolonnen bzw. Depopanisierungskolonnen bezeichnet werden. Dabei werden im hier verwendeten Sprachgebrauch unter "Demethanizern", "Deethanizern" und "Depropanizern" Anordnungen mit entsprechenden Rektifikationskolonnen verstanden, denen aber auch zusätzliche Apparate, beispielsweise Absorbern bei Deethanizern, zugeordnet sein können. Entsprechendes gilt, wenn davon die Rede ist, dass eine "Demethanisierung", eine "Deethanisierung" oder eine "Depropanisierung" vorgenommen wird. Ist nachfolgend davon die Rede, dass Fraktionen "vom Kopf" oder "aus dem Sumpf" von Demethanizern, Deethanizern und Depropanizern oder entsprechenden Rektifikationskolonnen abgezogen werden können, können diese auch alternativ oder zusätzlich zur Rektifikationskolonne vom Kopf bzw. aus dem Sumpf entsprechender zugeordneter Apparate abgezogen werden.

Gemäß einer ersten bevorzugten Ausgestaltung der vorliegenden Erfindung werden bei der Entfernung von Wasserstoff und Methan in dem oder den zweiten Vortrennschritten auch Ethan und Ethylen zumindest überwiegend entfernt, es wird also ein Deethanizer-First-Verfahren durchgeführt.

Dabei erfolgt die Entfernung von Wasserstoff und Methan in dem oder den zweiten Vortrennschritten, bei der auch Ethan und Ethylen zumindest überwiegend entfernt werden, unter Verwendung einer Deethanisierungskolonne. Auf einem sumpfnahen Trennboden einer derartigen Deethanisierungskolonne fällt in diesem Fall das vierte Komponentengemisch an, wobei unter einem "sumpfnahen Trennboden" ein Trennboden verstanden wird, der in der unteren Hälfte, insbesondere im unteren Drittel, im unteren Viertel oder im unteren Fünftel der Deethanisierungskolonne angeordnet ist. Eine entsprechende Flüssigkeit ist gegenüber dem zweiten Komponentengemisch an Wasserstoff, Methan sowie Kohlenwasserstoffen mit zwei Kohlenstoffatomen abgereichert bzw. wurden entsprechende Komponenten zu einem überwiegenden Anteil entfernt.

Besonders vorteilhaft ist es bei einer derartigen Ausgestaltung der vorliegenden Erfindung, das dritte Komponentengemisch zumindest teilweise in flüssigem Zustand im Bereich eines entsprechenden sumpfnahen Trennbodens, beispielsweise auf einen derartigen Trennboden oder oberhalb, der Deethanisierungskolonne einzuspeisen.

Dies ermöglicht es, den oder die ersten Vortrennschritte derart zu gestalten, dass in dem dritten Komponentengemisch Restgehalte an Wasserstoff sowie gegebenenfalls geringe Mengen an Methan und Kohlenwasserstoffe mit zwei Kohlenstoffatomen vorliegen können, weil diese in der Deethanisierungkolonne durch noch vorliegende Trennleistung im Bereich des sumpfnahen Trennbodens noch entfernt werden können. Dies ermöglicht eine vereinfachte Ausgestaltung des oder der ersten Vortrennschritte, weil diese nicht auf eine vollständige Entfernung von Wasserstoff sowie Methan und Kohlenwasserstoffe mit zwei Kohlenstoffatomen ausgelegt werden müssen.

Typischerweise wird im Rahmen der vorliegenden Erfindung aus dem Sumpf der Deethanisierungskolonne eine Sumpfflüssigkeit abgezogen und zumindest teilweise in eine Depropanisierungskolonne überführt. Diese Sumpfflüssigkeit weist typischerweise überwiegend oder ausschließlich Kohlenwasserstoffe mit drei und mehr Kohlenstoffatomen auf. In der Depropanisierungskolonne bzw. einem dieser zugeordneten Apparat wird eine Kopffraktion gebildet, die überwiegend oder ausschließlich Kohlenwasserstoffe mit drei Kohlenstoffatomen aufweist. Eine Sumpfflüssigkeit der Depropanisierungskolonne weist hingegen überwiegend oder ausschließlich schwerer siedende Kohlenwasserstoffe auf.

Das dritte Komponentengemisch kann dabei in einer Ausgestaltung der vorliegenden Erfindung zumindest teilweise, alternativ oder zusätzlich zu der Einspeisung in den Bereich des sumpfnahen Trennbodens der Deethanisierungskolonne, in flüssigem Zustand in den unteren Teil, insbesondere ebenfalls in der Depropanisierungskolonne eingespeist werden. Zwar kann in einem derartigen Fall typischerweise keine gezielte Entfernung von leichteren Komponenten wie Restwasserstoff aus dem dritten Komponentengemisch mehr erfolgen, jedoch kann in einem solchen Fall der eingebrachte Wasserstoff in besonders vorteilhafter Weise in einem stromabwärtig eingesetzten Hydrierungsverfahren, der eine vom Kopf der Depropanisierungskolonne oder einem dieser zugeordneten Apparat abgezogene Fraktion, die überwiegend oder ausschließlich Kohlenwasserstoffe mit drei Kohlenstoffatomen enthält, unterworfen wird, verwendet werden. Dies ermöglicht es, dort auf eine separate Einspeisung von zusätzlichem Wasserstoff zumindest teilweise zu verzichten. Wie auch nachfolgend erläutert, können eingebrachter Wasserstoff sowie andere leichtere Verbindungen, insbesondere Kohlenwasserstoffe mit zwei Kohlenstoffatomen, in einem stromab einer entsprechenden Hydrierung vorgesehenen Stripper ausgeschleust werden.

Die vom Kopf der Depropanisierungskolonne oder einem dieser zugeordneten Apparat abgezogene Fraktion, die überwiegend oder ausschließlich Kohlenwasserstoffe mit drei Kohlenstoffatomen enthält, und die gegebenenfalls anschließend hydriert wird, wird insbesondere im Zuge des oder der erwähnten ersten Trennschritte zur Gewinnung des ersten und zweiten Trennungsprodukts einem C3-Splitter zugeführt.

Wie mehrfach erwähnt, ist alternativ zu der soeben erläuterten ersten bevorzugten Ausgestaltung der Erfindung auch eine zweite vorteilhafte Ausgestaltung möglich, bei denen bei der Entfernung von Wasserstoff und Methan in dem oder den zweiten Vortrennschritten Ethan und Ethylen zumindest überwiegend nicht entfernt werden. Ethan und Ethylen gehen daher in diesen Ausführungsformen der Erfindung zumindest zum überwiegenden Teil in das vierte Komponentengemisch über. Es kommt also insbesondere ein Demethanizer-First-Verfahren zum Einsatz. Wird zur Entfernung von Wasserstoff und Methan dabei eine entsprechende Rektifikationskolonne eingesetzt, handelt es sich bei dem vierten Komponentengemisch insbesondere um eine Sumpfflüssigkeit einer derartigen Rektifikationskolonne.

Die Entfernung von Wasserstoff und Methan in dem oder den zweiten Vortrennschritten, bei der Ethan und Ethylen zumindest überwiegend nicht entfernt werden, kann also unter Verwendung einer Demethanisierungskolonne durchgeführt werden. Einer derartigen Demethanisierungskolonne oder einem dieser zugeordndeten Apparat wird kopfseitig ein überwiegend oder ausschließlich Wasserstoff und Methan enthaltendes Komponentengemisch entnommen, aus dem Sumpf kann eine überwiegend oder ausschließlich Kohlenwasserstoffe mit zwei und mehr Kohlenstoffatomen enthaltende Sumpfflüssigkeit abgezogen werden. Diese Sumpfflüssigkeit kann insbesondere anschließend einer Deethanisierung unterworfen werden, wie auch nachfolgend noch erläutert.

In der soeben erläuterten Ausgestaltung der vorliegenden Erfindung kann das dritte Komponentengemisch insbesondere zumindest teilweise in flüssigem Zustand in einen unteren Teil, insbesondere einen Bereich eines sumpfnahen Trennbodens im oben erläuterten Sinn, der Demethanisierungskolonne eingespeist werden. Grundsätzlich ergibt sich hieraus derselbe Vorteil wie bereits zuvor unter Bezugnahme auf das Deethanizer-First-Verfahren erläutert, nämlich, dass enthaltener Restwasserstoff und ggf. Methan in der Demethanisierungskolonne noch ausgeschleust werden kann. Dies ermöglicht es auch in dieser Verfahrensvariante, den oder die ersten Vortrennschritte derart zu gestalten, dass in dem dritten Komponentengemisch Restgehalte an Wasserstoff vorliegen können, weil diese in der Demethanisierungkolonne noch entfernt werden können. Dies ermöglicht eine vereinfachte Ausgestaltung des oder der ersten Vortrennschritte, weil diese nicht auf eine vollständige Entfernung von Wasserstoff ausgelegt werden müssen und daher einfacher ausgestaltet werden können.

Wie bereits erwähnt, kann aus dem Sumpf der Demethanisierungskolonne eine Sumpfflüssigkeit abgezogen und zumindest teilweise in eine Deethanisierungskolonne überführt werden. Einer derartigen Deethanisierungskolonne in einem Demethanizer-First-Verfahren oder einem dieser zugeordneten Apparat kann kopfseitig ein überwiegend oder ausschließlich Kohlenwasserstoffe mit zwei Kohlenstoffatomen enthaltendes Komponentengemisch, das nachfolgend typischerweise in einen C2-Splitter überführt, also dem oder den zweiten Trennschritten unterworfen wird, entnommen werden. Ferner kann der Deethanisierungskolonne in einem Demethanizer-First-Verfahren eine Sumpfflüssigkeit entnommen werden, die überwiegend oder ausschließlich Kohlenwasserstoffe mit drei und mehr Kohlenstoffatomen enthält. Gemäß einer Ausführungsform der vorliegenden Erfindung kann auch vorgesehen sein, das dritte Komponentengemisch zumindest teilweise in einen Bereich eines sumpfnahen Trennbodens dieser Deethanisierungskolonne einzuspeisen.

Aus dem Sumpf der Deethanisierungskolonne kann dabei vorteilhafterweise eine Sumpfflüssigkeit abgezogen und zumindest teilweise in eine Depropanisierungskolonne überführt werden. Zur Betriebsweise und den Merkmalen einer derartigen Depropanisierungskolonne und die jeweils erzielbaren Vorteile sei auf die obigen Erläuterungen verwiesen.

Wie bereits zuvor erwähnt, kann das dritte Komponentengemisch zumindest teilweise in der Depropanisierungskolonne eingespeist werden. Dies gilt in gleicher Weise für ein Deethanizer-First-Verfahren und ein Demethanizer-First-Verfahren. In beiden Fällen kann eine Propan und Propylen enthaltende Fraktion vom Kopf der Depropanisierungskolonne oder eines zugeordneten Apparats abgezogen und unter Zugabe von Wasserstoff und unter Erhalt einer hydrierten Fraktion zumindest teilweise einer Hydrierung unterworfen werden.

Wie ebenfalls bereits zuvor angesprochen, können nach der Hydrierung leichter als Propan und Propylen siedende Komponenten aus der hydrierten Fraktion zumindest teilweise ausgetrieben werden. Auf diese Weise ist es möglich, in Form des dritten Komponentengemischs eingespeiste Komponenten wieder zu entfernen. Hierzu ist kein nachteiliger oder unverhältnismäßiger Zusatzaufwand erforderlich, da ein entsprechendes Austreiben von Wasserstoff stromab einer typischerweise erfolgenden Hydrierung ohnehin vorgenommen wird und daher auch die anderen leichten Komponenten ausgetrieben werden können.

Bei einem Depropanizer-First-Verfahren, mit dem die vorliegende Erfindung ebenfalls zum Einsatz kommen kann, kann die Einspeisung des dritten Komponentengemischs in einer dieser Depropanisierungskolonne nachgeordneten Rektifikationskolonne erfolgen.

Unter dem Begriff "Strippen" bzw. "Austreiben" sei dabei im Rahmen der vorliegenden Anmeldung ein Trennschritt verstanden, der ein Abreichern eines Komponentengemischs an leichten Komponenten durch Temperaturerhöhung und/oder Durchströmen mit einem Strip- bzw. Austreibgas umfasst. Zum Strippen kommt dabei zweckmäßigerweise eine Stripkolonne, kurz "Stripper", zum Einsatz, die über entsprechend eingerichtete Mittel verfügt, beispielsweise über eine Heizeinrichtung und/oder eine Einspeisemöglichkeit für Stripgas. Ebenso kann eine Stripkolonne einen Kondensator aufweisen und damit sehr ähnlich aufgebaut bzw. ausgerüstet sein wie eine Rektifikationskolonne.

Insbesondere wird im Rahmen der vorliegenden Erfindung im Rahmen des oder der ersten Vortrennschritte des ersten Komponentengemischs dessen Wasserstoffgehalt auf einen Wert von 0 bis 10 mol%, insbesondere 0,1 bis 5 mol%, beispielsweise 0,2 bis 2 mol% abgereichert. Bei derartigen Wasserstoffgehalten kann das zweite Komponentengemisch, da es in seiner sonstigen Zusammensetzung ausreichend ähnlich einem entsprechenden Fluid aus einem Dampfspaltverfahren ist, der gemeinsamen Trennung zugeführt werden. Wie erwähnt, kann hierbei noch vorhandener Wasserstoff einfach entfernt werden.

Wie erwähnt, umfassen der oder die ersten Vortrennschritte, denen das erste Komponentengemisch unterworfen wird, ferner eine Druckerhöhung, die insbesondere auf einen Absolutdruck von 3 bis 40 bar, insbesondere von 10 bis 30 bar, beispielsweise von 12 bis 30 bar, erfolgt. Das Druckniveau richtet sich dabei nach einem Druckniveau, auf der ein Demethanizer oder Deethanizer oder Depropanizer, wie er in dem oder den zweiten Vortrennschritten eingesetzt wird, betrieben wird, also die dort ebenfalls vorgenommene Druckerhöhung im Rahmen des oder der zweiten Vortrennschritte erfolgt. Daher können auf diese Weise die Druckniveaus des dritten und vierten Komponentengemischs in besonders vorteilhafter Weise aneinander angeglichen werden.

Die Wasserstoffabreicherung im Rahmen der Vorbehandlung des ersten Komponentengemischs oder dessen der Vorbehandlung unterworfenen Teils kann insbesondere eine Teilkondensation von Kohlenwasserstoffen mit drei Kohlenstoffatomen nach der erläuterten Druckerhöhung bzw. Verdichtung umfassen. Auf diese Weise wird eine Fraktion gebildet, die überwiegend Kohlenwasserstoffe mit drei Kohlenstoffatomen enthält, in die jedoch auch die anderen genannten Komponenten teilweise übergehen können. Diese Fraktion ist jedenfalls gegenüber dem ersten Komponentengemisch an Wasserstoff abgereichert. Eine entsprechende Kondensation ist besonders vorteilhaft, weil diese im Rahmen der vorliegenden Erfindung, wie nachfolgend erläutert, zumindest teilweise unter Verwendung von Kälte durchgeführt werden kann, welche durch in dem Verfahren vorhandene Prozessströme bereitgestellt werden kann.

Insbesondere kann die Teilkondensation unter Verwendung von Kälte durchgeführt werden, die zumindest teilweise durch Entspannen eines überwiegend oder ausschließlich Propan enthaltenden Stroms gewonnen werden kann. Bei diesem überwiegend oder ausschließlich Propan enthaltenden Strom kann es sich beispielsweise um das zweite Trennungsprodukt handeln, das in dem oder den ersten Trennschritten gebildet wird. Dieses zweite Trennungsprodukt kann zur Erzeugung von Kälte entspannt und anschließend in den Prozess zurückgeführt werden, insbesondere in das Verfahren zur Propandehydrierung oder das Dampfspaltverfahren.

Ferner ist es auch möglich, die Teilkondensation unter Verwendung von Kälte durchzuführen, die zumindest teilweise durch Entspannen eines Teils des ersten Komponentengemischs oder dessen dem oder den ersten Vortrennschritten unterworfenen Anteils zu erzeugen. Beispielsweise kann das erste Komponentengemisch oder dessen dem oder den ersten Vortrennschritten unterworfener Anteil dem oder den ersten Vortrennschritten in Form eines Stoffstroms zugeführt werden, der verdichtet wird, und von dem ein Teilstrom stromab der Verdichtung entspannt wird. Der entspannte Teilstrom kann der Verdichtung erneut zugeführt werden, so dass auf diese Weise kontinuierlich Kälte generiert werden kann. Auch sogenannte Coldboxverfahren, wie sie grundsätzlich aus dem Stand der Technik bekannt sind, oder auf anderen Trennprinzipien beruhende Verfahren können im Rahmen der vorliegenden Erfindung eingesetzt werden.

Besonders vorteilhaft ist es, wenn das Verfahren zur Propandehydrierung unter wasserfreien und/oder in kompletter Abwesenheit von Sauerstoff (auch in kovalent gebundener Form und/oder während der Regenerierung) durchgeführt wird. Auf diese Weise wird es möglich, das erste Komponentengemisch derart zu bilden, dass sich in ihm weder Wasser noch sauerstoffhaltige Verbindungen, insbesondere Kohlendioxid, wiederfinden. Auf diese Weise ist es besonders einfach möglich, ein entsprechendes erstes Komponentengemisch dem oder den ersten Vortrennschritten und insbesondere anschließend dem oder den ersten Trennschritten zuzuführen, weil keine Abtrennung dieser Komponenten erforderlich ist. Das erste Komponentengemisch kann, mit anderen Worten, ohne Abtrennung von beispielsweise Wasser und Kohlendioxid bei der Bildung des dritten Komponentengemischs, beispielsweise einer Rektifikationskolonne zugeführt werden, die der Deethanisierung dient, und die typischerweise bei tiefkalten Temperaturen betrieben wird, bei denen Wasser und Kohlendioxid ausfrieren würden.

Wie erwähnt, ist die Zuführung des dritten Komponentengemischs nach dem oder den ersten Vortrennschritten zu dem oder den ersten Trennschritten, in dem oder in denen das dritte Komponentengemisch mit dem vierten Komponentengemisch bzw. einem hieraus gebildeten Komponentengemisch vereinigt wird, dann besonders vorteilhaft, wenn sich die jeweiligen Zusammensetzungen gleichen bzw. um nicht mehr als einen vorbestimmten Umfang unterscheiden.

Es ist daher besonders vorteilhaft, wenn ein Wasserstoffgehalt in dem dritten Komponentengemisch um nicht mehr als 50%, insbesondere um nicht mehr als 25%, beispielsweise um nicht mehr als 10%, von einem Wasserstoffgehalt in dem vierten Komponentengemisch abweicht, und wenn ein Gehalt an Kohlenwasserstoffen mit drei Kohlenstoffatomen, insbesondere Propylen, in dem dritten Komponentengemisch um nicht mehr als 50%, insbesondere um nicht mehr als 25%, beispielsweise um nicht mehr als 10%, von einem Gehalt an Kohlenwasserstoffen mit drei Kohlenstoffatomen, insbesondere Propylen, in dem vierten Komponentengemisch abweicht.

Die vorliegende Erfindung erstreckt sich ferner auf eine Anlage zur Herstellung von Propylen, mit einer ersten Reaktoreinheit, die zum Durchführen eines Verfahrens zur Propandehydrierung unter Erhalt eines ersten Komponentengemischs, das zumindest Wasserstoff, Ethan, Ethylen, Propan und Propylen enthält, bereitgestellt und eingerichtet ist, einer zweiten Reaktoreinheit, die zum Durchführen eines Dampfspaltverfahrens unter Erhalt eines zweiten Komponentengemischs, das zumindest Wasserstoff, Methan, Ethan, Ethylen, Propan und Propylen enthält, bereitgestellt und eingerichtet ist, einer ersten Trenneinheit, die zum Bilden eines ersten Trennungsprodukts, das zumindest überwiegend Propylen enthält, unter Verwendung zumindest eines Teils des Propylens des ersten und des zweiten Komponentengemischs und unter Einsatz eines oder mehrerer erster Trennschritte bereitgestellt und eingerichtet ist, wobei die erste Trenneinheit ferner zum Bilden eines zweiten Trennungsprodukts, das zumindest überwiegend Propan enthält, unter Verwendung zumindest eines Teils des Propans des ersten und des zweiten Komponentengemischs und unter Einsatz des oder der ersten Trennschritte bereitgestellt und eingerichtet ist, und einer zweiten Trenneinheit, die zum Bilden eines dritten Trennungsprodukts, das zumindest überwiegend Ethylen enthält, unter Verwendung zumindest eines Teils des Ethylens des ersten und des zweiten Komponentengemischs und unter Einsatz eines oder mehrerer zweiter Trennschritte bereitgestellt und eingerichtet ist, wobei die zweite Trenneinheit ferner zum Bilden eines vierten Trennungsprodukts, das zumindest überwiegend Ethan enthält, unter Verwendung zumindest eines Teils des Ethans des ersten und des zweiten Komponentengemischs und unter Einsatz des oder der zweiten Trennschritte bereitgestellt und eingerichtet ist.

Erfindungsgemäß zeichnet sich eine derartige Anlage aus durch eine erste Vortrenneinheit, die dafür bereitgestellt und eingerichtet ist, zumindest einen Teil des ersten Komponentengemischs unter Erhalt eines dritten Komponentengemischs einem oder mehreren ersten Vortrennschritten zu unterwerfen, der oder die eine Druckerhöhung und eine zumindest teilweise Entfernung von Wasserstoff umfassen, eine zweite Vortrenneinheit, die dafür bereitgestellt und eingerichtet ist, zumindest ein Teil des zweiten Komponentengemischs unter Erhalt eines vierten Komponentengemischs einem oder mehreren zweiten Vortrennschritten zu unterwerfen, der oder die eine Druckerhöhung, eine zumindest teilweise Entfernung von Wasserstoff und eine zumindest teilweise Entfernung von Methan umfassen, und Mittel, die dafür bereitgestellt und eingerichtet sind, zumindest einen Teil des dritten Komponentengemischs gemeinsam mit zumindest einem Teil des vierten Komponentengemischs der ersten Trenneinheit zuzuführen und dem oder den ersten Trennschritten zu unterwerfen.

Zu Merkmalen und Vorteilen der erfindungsgemäß vorgeschlagenen Anlage sei auf die zuvor bereits bezüglich des erläuterten Prozesses und seiner vorteilhaften Ausgestaltung in erläuterten Merkmale und Vorteile ausdrücklich verwiesen. Entsprechendes gilt insbesondere für eine Anlage gemäß einer besonders bevorzugten Ausführungsformen der vorliegenden Erfindung, welche Mittel aufweist, die zur Durchführung eines entsprechenden Verfahrens eingerichtet sind.

Die Erfindung erstreckt sich ferner auf ein Verfahren zum Umrüsten einer Anlage, die zur Durchführung eines Dampfspaltverfahrens unter Verwendung einer Anzahl von Anlagenkomponenten wie Spaltöfen, Aufbereitungseinrichtungen und Trennapparaten eingerichtet ist, wobei der Anlage vor der Umrüstung ein Kohlenwasserstoffe enthaltendes Einsatzgemisch mit einer ersten Zusammensetzung zugeführt wird. Die Umrüstung umfasst erfindungsgemäß, der Anlage anstelle des Kohlenwasserstoffe enthaltenden Einsatzgemischs mit der ersten Zusammensetzung ein Kohlenwasserstoffe enthaltendes Einsatzgemisch mit einer zweiten, abweichenden Zusammensetzung zuzuführen, und eine oder mehrere der Anlagenkomponenten anstatt für das Dampfspaltverfahren für ein Verfahren zur Propandehydrierung zu nutzen, also freiwerdende Kapazitäten entsprechend umzuwidmen.

Beispielhaft sei hier eine Änderung des Einsatzgemisches des Dampfspaltverfahrens von schwereren Kohlenwasserstoffen, beispielsweise überwiegend Naphta, hin zu leichteren Kohlenwaserstoffen, beispielsweise Ethan und/oder Propan sowie Butan, zu nennen. Während gewisse Anlagenteile zur Verarbeitung des gesamten Produktgases, wie beispielsweise der Rohgasverdichter, sowie Anlagenteile zur Verarbeitung der leichten Produktfraktion, wie beispielsweise der Demethanizer, nach der Änderung des Einsatzgemisches voraussichtlich gleich stark oder sogar höher als zuvor belastet sind, werden andere Anlagenteile, beispielsweise zur Verarbeitung von schwereren Produktfraktionen, entlastet. Diese entlasteten Anlagenteile können den Depropanizer sowie sämtliche Anlagenteile zur Verarbeitung einer Fraktion mit Kohlenwasserstoffen mit drei Kohlenstoffatomen einschließlich einer Hydrierung und eines Splitters, umfassen. Diese Anlagenteile können dann zusätzlich für ein Verfahren zur Propandehydrierung genutzt werden. Hierbei ist die in der vorliegenden Erfindung beschriebene Vorgehensweise der vorangehenden Wasserstoffentfernung aus dem Produktgas der Propandehydrierung insbesondere von Vorteil, da durch diese Vorgehensweise die stark ausgelasteten bestehenden Anlagenteile des Dampfspaltverfahrens, wie beispielsweise der Demethanizer, nicht noch stärker belastet werden.

Erfindungsgemäß umfasst eine entsprechende Umrüstung, ein Verfahren durchzuführen, wie es zuvor beschrieben wurde, und/oder eine entsprechende Anlage bereitzustellen. Auf diese Weise kann der bereits eingangs erwähnte Vorteil erzielt werden, dass entsprechende Produkte einer Propandehydrierung zusammen mit den Produkten des Dampfspaltverfahrens aufzureinigen und auf eine separate Aufreinigung zu verzichten.

Die Erfindung wird nachfolgend unter Bezugnahme auf die beigefügten Zeichnungen näher erläutert, in der eine bevorzugte Ausführungsformen der vorliegenden Erfindung gegenüber dem Stand der Technik erläutert ist.

### Kurze Beschreibung der Zeichnungen

Figur 1 veranschaulicht einen gemäß einer Ausführungsform der Erfindung ausgestalteten Prozess in stark vereinfachter, schematischer Darstellung.
Figur 2 veranschaulicht einen gemäß einer Ausführungsform der Erfindung ausgestalteten Prozess in vereinfachter schematischer Darstellung.
Figur 3 veranschaulicht Prozessalternativen gemäß Ausführungsformen der Erfindung sowie nicht erfindungsgemäße Prozessalternativen.
Figur 4 setzt die Darstellung der Figur 3 fort.

In den Figuren sind einander baulich und/oder funktionell entsprechende Elemente mit identischen Bezugszeichen angegeben und werden der Übersichtlichkeit halber nicht wiederholt erläutert.

### Ausführliche Beschreibung der Zeichnungen

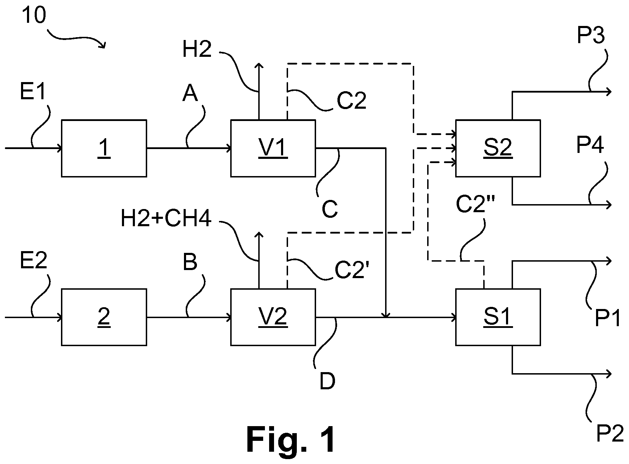

In Figur 1 ist ein erfindungsgemäß ausgestalteter Prozess in stark vereinfachter, schematischer Darstellung veranschaulicht und insgesamt mit 10 bezeichnet.

Der Prozess 10 umfasst ein Verfahren 1 zur Propandehydrierung, ein Dampfspaltverfahren 2, einen oder mehrere erste Vortrennschritte V1, einen oder mehrere zweite Vortrennschritte V2, einen oder mehrere erste Trennschritte S1 sowie einen oder mehrere zweite Trennschritte S2. Der oder die ersten Vortrennschritte V1, der oder die zweiten Vortrennschritte V2, der oder die ersten Trennschritte S1 und der oder die zweiten Trennschritte S2 können dabei jeweils beliebig gruppiert und beispielsweise in entsprechenden Anlagenkomponenten zusammengefasst sein. Ein wesentlicher Aspekt des Prozesses 10 ist jedoch, dass der oder die ersten Vortrennschritte V1 und der oder die zweiten Vortrennschritte V2 jeweils getrennt voneinander ablaufen bzw. durchgeführt werden, d.h. dass Komponentengemische, die dem oder den ersten Vortrennschritten zugeführt werden, zumindest nicht an derselben Stelle wie andere Komponentengemische dem oder den zweiten Vortrennschritten zugeführt werden und umgekehrt.

Dem Verfahren 1 zur Propandehydrierung wird im dargestellten Beispiel ein erster Einsatzstrom E1 zugeführt, der insbesondere Propan umfassen kann. Das Verfahren 1 zur Propandehydrierung wird in grundsätzlich bekannter Weise durchgeführt, so dass in diesem ein erstes Komponentengemisch A gebildet wird, das zumindest Wasserstoff, Ethan, Ethylen, Propan und Propylen enthält, und das aus dem Verfahren 1 zur Propandehydrierung in Form eines entsprechenden Stoffstroms ausgeführt werden kann. Das Verfahren 1 zur Propandehydrierung kann insbesondere unter Verwendung eines oder mehrerer geeigneter Reaktoren, die in fachüblicher Weise ausgebildet sein können, durchgeführt werden.

Das erste Komponentengemisch A bzw. der entsprechende Stoffstrom wird im dargestellten Beispiel zumindest teilweise dem oder den ersten Vortrennschritten V1 zugeführt, in dem oder denen das erste Komponentengemisch A bzw. der entsprechende Stoffstrom einer Druckerhöhung und einer zumindest teilweisen Entfernung von Wasserstoff unterworfen wird. Wie oben erwähnt, kann dies in grundsätzlich bekannter Weise erfolgen. Insbesondere kann das erste Komponentengemisch A bzw. der entsprechende Stoffstrom in dem oder den ersten Vortrennschritten V1 verflüssigt werden. Abgetrennter Wasserstoff ist in Form eines mit H2 bezeichneten Stoffstroms veranschaulicht. Auch eine zumindest teilweise Entfernung von Kohlenwasserstoffen mit zwei Kohlenstoffatomen ist möglich, aber optional, wie mit einem gestrichelt dargestellten Stoffstrom C2 veranschaulicht. Auf diese Weise wird ein Komponentengemisch erhalten, das hier auch als drittes Komponentengemisch C bezeichnet wird, und das in Form eines entsprechenden Stoffstroms aus dem oder den ersten Vortrennschritten V1 ausgeführt werden kann. Mögliche Wasserstoffgehalte des dritten Komponentengemischs C bzw. des entsprechenden Stoffstroms wurden bereits oben erläutert. Insbesondere enthält das dritte Komponentengemisch C stromab des oder der ersten Vortrennschritte V1 noch Kohlenwasserstoffe mit drei Kohlenstoffatomen sowie geringere Mengen an anderen Komponenten, beispielsweise Kohlenwasserstoffe mit zwei Kohlenwasserstoffatomen, soweit noch nicht entfernt, und Kohlenwasserstoffe mit vier Kohlenstoffatomen, die in dem Verfahren 1 zur Propandehydrierung als Nebenprodukte gebildet werden.

Sofern im ersten Komponentengemisch A auch andere Komponenten wie Wasser und Kohlendioxid enthalten sind, können diese auch in dem oder den ersten Vortrennschritten V1 entfernt werden.

Dem Dampfspaltverfahren 2, das ebenfalls in fachüblicher Weise, beispielsweise auch unter Verwendung mehrerer Spaltöfen, durchgeführt werden kann, wird ein kohlenwasserstoffreicher Einsatz in Form eines Stoffstroms E2 zugeführt. Der kohlenwasserstoffreiche Einsatz kann insbesondere Naphtha und leichtere Kohlenwasserstoffe, aber auch schwere Kohlenwasserstoffe, umfassen. Insbesondere kann der kohlenwasserstoffreiche Einsatz auch paraffinische Kohlenwasserstoffe mit zwei, drei und vier Kohlenstoffatomen umfassen, insbesondere Ethan, Propan und Butan. Dem Dampfspaltverfahren 2 insgesamt oder unterschiedlichen Spaltöfen, die in dem Dampfspaltverfahren 2 eingesetzt werden, können auch unterschiedliche Kohlenwasserstoffeinsätze zugeführt und dort unter unterschiedlichen Spaltbedingungen bearbeitet werden. In dem Dampfspaltverfahren 2 werden die Kohlenwasserstoffe des oder der kohlenwasserstoffreichen Einsätze zumindest teilweise umgesetzt, so dass ein zweites Komponentengemisch B erhalten wird, das zumindest Wasserstoff, Methan, Ethan, Ethylen, Propan und Propylen enthält. Das zweite Komponentengemisch B kann aus dem Dampfspaltverfahren 2 in Form eines entsprechenden Stoffstroms abgezogen und anschließend zumindest zum Teil dem einem oder den mehreren zweiten Vortrennschritten V2 zugeführt werden. Die Zusammensetzung des zweiten Komponentengemischs B bzw. des entsprechenden Stoffstroms richtet sich maßgeblich nach dem dem Dampfspaltverfahren 2 zugeführten kohlenwasserstoffreichen Einsatz.

Wie bereits mehrfach erläutert, kann bzw. können der oder die zweiten Vortrennschritte V2 insbesondere eine Demethanisierung oder eine Deethanisierung umfassen. Bei beiden Möglichkeiten erfolgt eine Druckerhöhung. Bei einer Demethanisierung erfolgt eine zumindest teilweise Entfernung von Wasserstoff und eine zumindest teilweise Entfernung von Methan aus dem zweiten Komponentengemisch B, wie in Form eines mit H2+CH4 bezeichneten Stoffstroms veranschaulicht. Ethan und Ethylen bzw. allgemein Kohlenwasserstoffe mit zwei Kohlenstoffatomen werden hierbei jedoch nicht aus dem zweiten Komponentengemisch B entfernt. Im Gegensatz dazu erfolgt bei einer Deethanisierung ebenfalls eine zumindest teilweise Entfernung von Wasserstoff und Methan, so dass auch für diesen Fall der mit H2+CH4 bezeichnete Stoffstrom gebildet wird, zudem jedoch eine zumindest teilweise Entfernung von Kohlenwasserstoffen mit zwei Kohlenstoffatomen. Letzteres ist mit dem gestrichelt dargestellten Stoffstrom C2' veranschaulicht. Bei einem Deethanizer-First-Verfahren verlässt der mit H2+CH4 bezeichnete Stoffstrom den Kopf einer Demethanisierungskolonne, die der Deethanisierungskolonne nachgeschaltet ist, oder einem der Demethanisierungskolonne zugeordneten Apparat, und der Stoffstrom C2' deren Sumpf. Bei einem Demethanizer-First-Verfahren, bei dem kein entsprechender Stoffstrom C2' gebildet wird, verlässt der mit H2+CH4 bezeichnete Stoffstrom den Kopf einer Demethanisierungskolonne oder einen dieser zugeordneten Apparat, in deren Sumpf liegt hingegen ein Komponentengemisch vor, das neben Kohlenwasserstoffe mit zwei Kohlenstoffatomen auch noch schwere Kohlenwasserstoffe enthält, so dass der Stoffstrom C2' nicht aus dem Sumpf der Demethanisierungskolonne abgezogen werden kann. Dieser wird vom Kopf einer der Demethanisierungskolonne nachgeschalteten Deethanisierungskolonne oder einem einer Deethanisierungskolonne zugeordneten Apparat abgezogen.

Ungeachtet des spezifisch durchgeführten Verfahrens wird jedoch in beiden Fällen durch die Verwendung des oder der zweiten Vortrennschritte ein Komponentengemisch D gebildet, das hier als viertes Komponentengemisch bezeichnet wird, und das gegenüber dem zweiten Komponentengemisch B zumindest an Wasserstoff und an Methan abgereichert ist bzw. das dadurch gebildet wird, dass zumindest Wasserstoff von Methan zumindest teilweise aus dem zweiten Komponentengemisch B entfernt werden.

Dieses vierte Komponentengemisch kann physisch in Form eines entsprechenden Stoffstroms aus dem oder den zweiten Vortrennschritten V2 abgezogen werden. Die vorliegende Erfindung umfasst jedoch auch, dass ein derartiges viertes Komponentengemisch D in einer in dem oder den zweiten Vortrennschritten V2 verwendeten Einrichtung vorliegt. Beispielsweise kann es sich bei dem vierten Komponentengemisch D, wie mehrfach erläutert, um eine Flüssigkeit imBereich eines sumpfnahen Trennbodens einer Demethanisierungs- oder Deethanisierungskolonne handeln, je nachdem ob ein Deethanizer-First-Verfahren oder ein Demethanizer-First-Verfahren eingesetzt wird. Auch diese Flüssigkeit ist gegenüber dem zweiten Komponentengemisch B an den zuvor erläuterten Komponenten (Wasserstoff, Methan und gegebenenfalls Kohlenwasserstoffen mit zwei Kohlenstoffatomen) abgereichert bzw. wurde dieses dadurch gebildet, dass entsprechende Komponenten zumindest teilweise aus dem zweiten Komponentengemisch B entfernt wurden.

Wie auch nachfolgend erläutert, kann dem Bereich des sumpfnahen Trennbodens einer entsprechenden Demethanisierungs- oder Deethanisierungskolonne das bereits erwähnte dritte Komponentengemisch C zugespeist werden, so dass aus dem Sumpf der Demethanisierungs- oder Deethanisierungskolonne nicht das vierte Komponentengemisch D sondern ein weiteres Komponentengemisch, das sich durch die Vereinigung des dritten Komponentengemischs C mit dem vierten Komponentengemisch D und die noch in geringem Umfang vorhandene Trennwirkung im Bereich des sumpfnahen Trennbodens der Demethanisierungs- oder Deethanisierungskolonne ergibt, abgezogen wird. Eine Zuspeisung des dritten Komponentengemischs C kann jedoch auch statt in den Bereich des sumpfnahen Trennbodens der Demethanisierungs- oder Deethanisierungskolonne erst stromab hiervon erfolgen.

In sämtlichen erläuterten Fällen werden jedoch im Zuge des Prozesses 10 der oder die ersten Trennschritte S1 durchgeführt, in dem oder denen zwei Trennungsprodukte gebildet werden, nämlich ein erstes Trennungsprodukt P1 und ein zweites Trennungsprodukt P2. Das erste Trennungsprodukt P1 umfasst dabei überwiegend oder ausschließlich Propylen, das zweite Trennungsprodukt P2 überwiegend oder ausschließlich Propan. Das zweite Trennungsprodukt P2 kann insbesondere in den Prozess 10 zurückgeführt werden. Das erste Trennungsprodukt P1 stellt hingegen eines der Produkte des Prozesses 10 dar.

Das Propylen des ersten Trennungsprodukts P1 und das Propan des zweiten Trennungsprodukts P2 stammen dabei jeweils zumindest teilweise sowohl aus dem ersten Komponentengemisch A und dem zweiten Komponentengemisch B und damit auch jeweils zumindest teilweise sowohl aus dem dritten Komponentengemisch C und dem vierten Komponentengemisch D. Mit anderen Worten schlägt die vorliegende Erfindung eine zumindest teilweise vereinigte Gewinnung des ersten Trennungsprodukts P1 und des zweiten Trennungsprodukts P2 aus einem aus einem Verfahren 1 zur Propandehydrierung stammenden ersten Komponentengemisch A und aus einem aus einem Dampfspaltverfahren 2 stammenden zweiten Komponentengemisch B bzw. aus hieraus gebildeten dritten und vierten Komponentengemischen C und D vor.

Die ersten Trennschritte S1 umfassen dabei typischerweise in letzter Stufe, wie bereits mehrfach erläutert, die Verwendung eines C3-Splitters, dem ein Gemisch von überwiegend oder ausschließlich Kohlenwasserstoffen mit drei Kohlenstoffatomen, insbesondere von Propylen und Propan, zugeführt werden. Stromauf eines entsprechenden C3-Splitters kann eine entsprechende Hydrierung vorgesehen sein, wie ebenfalls bereits erwähnt. Wiederum stromauf dieser Hydrierung, die optional auch weggelassen werden kann, kann insbesondere eine Depropanisierung vorgesehen sein, die unter Verwendung einer Depropanisierungskolonne durchgeführt werden kann. Einer entsprechenden Depropanisierungskolonne oder einem der Depropanisierungskolonne zugeordneten Apparat kann kopfseitig ein Komponentengemisch entnommen werden, das überwiegend oder ausschließlich Kohlenwasserstoffe mit drei Kohlenstoffatomen enthält, und sumpfseitig ein Komponentengemisch, das schwerere Kohlenwasserstoffe enthält. Das kopfseitig der Depropanisierungskolonne oder dem zugeordneten Apparat entnommene Komponentengemisch kann, gegebenenfalls nach der erwähnten optionalen Hydrierung, dem C3-Splitter zugeführt werden.

Wiederum der Depropanisierung können eine Demethanisierung und eine Deethanisierung (bei einem Demethanizer-First-Verfahren) oder eine Deethanisierung (bei einem Deethanizer-First-Verfahren) in der jeweils angegebenen Reihenfolge vorangehen, wobei der gemäß diesen Alternativen jeweils erste Trennschritt (Demethanisierung in dem Demethanizer-First-Verfahren bzw. Deethanisierung in dem Deethanizer-First-Verfahren) in der hier verwendeten Systematik zumindest zum Teil einen Teil des oder der zweiten Vortrennschritte V2 bzw. einen der hier verwendeten Trennschritte darstellt.

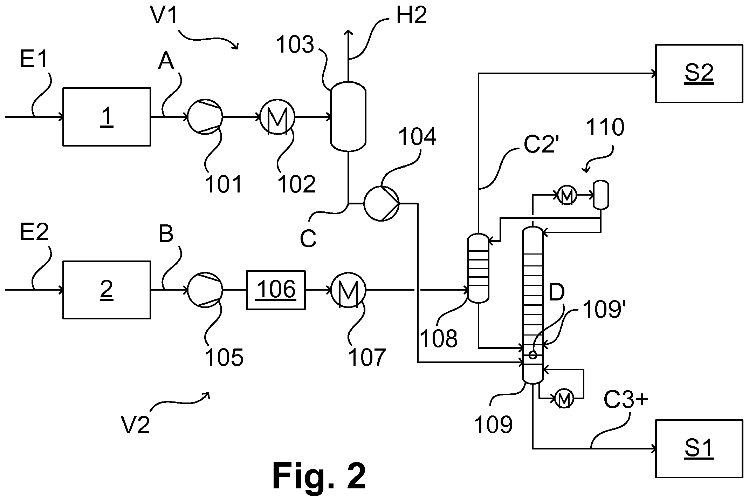

In beiden Fällen wird dabei einem Sumpf einer in der Deethanisierung eingesetzten Deethanisierungskolonne ein Komponentengemisch entnommen, das überwiegend oder ausschließlich Kohlenwasserstoffe mit drei und mehr Kohlenstoffatomen enthält. Dieses wird jeweils der Depropanisierung zugeführt. Weitere Details sind in der nachfolgenden Figur 2 erläutert. Bei einem Demethanizer-First-Verfahren wird der Deethanisierung ein Sumpfprodukt der Demethanisierung teilweise oder vollständig zugeführt, das lediglich an Wasserstoff und Methan abgereichert bzw. von diesen befreit ist, das aber noch Kohlenwasserstoffe mit zwei und mehr Kohlenstoffatomen enthält. Bei einem Deethanizer-First-Verfahren wird der Demethanisierung ein Kopfprodukt der Deethanisierung teilweise oder vollständig zugeführt, das Kohlenwasserstoffe mit zwei und weniger Kohlenstoffatomen sowie Wasserstoff enthält, ein Sumpfprodukt der Deethanisierung, das Kohlenwasserstoffe mit drei und mehr Kohlenstoffatomen enthält, wird hingegen teilweise oder vollständig der Depropanisierung zugeführt.

Das dritte Komponentengemisch C kann dabei insbesondere dem Bereich eines sumpfnahen Bodens einer Demethanisierungskolonne, dem Bereich eines sumpfnahen Bodens einer Deethanisierungskolonne oder einer Depropanisierungskolonne zugespeist werden wie bereits oben ausführlich erläutert. An der jeweiligen Einspeisestelle beginnt die "gemeinsame" Trennung, die die vorliegende Erfindung vorschlägt.

Ferner umfasst der Prozess 10 einen oder mehrere zweite Trennschritte S2, in dem oder denen ein drittes Trennungsprodukt P3 und ein viertes Trennungsprodukt P4 gebildet werden. Das dritte Trennungsprodukt P3 umfasst überwiegend oder ausschließlich Ethylen, das vierte Trennungsprodukt P4 umfasst hingegen überwiegend oder ausschließlich Ethan.

Sowohl das Ethylen des dritten Trennungsprodukts P3 als auch das Ethan des vierten Trennungsprodukts P4 stammen dabei jeweils zumindest teilweise sowohl aus dem ersten Komponentengemisch A als auch aus dem zweiten Komponentengemisch B und damit ebenfalls zumindest teilweise sowohl aus dem dritten Komponentengemisch C als aus dem vierten Komponentengemisch D. Insbesondere das vierte Trennungsprodukt P4 kann in dem Prozess 10 zurückgeführt werden.

Insbesondere können der oder die zweiten Trennschritte die Verwendung eines C2-Splitters umfassen, in den ein Gemisch aus überwiegend oder ausschließlich Kohlenwasserstoffen mit zwei Kohlenstoffatomen eingespeist werden kann, insbesondere ein Gemisch aus Ethylen und Ethan. Ein derartiges Gemisch kann insbesondere bei einem Deethanizer-First-Verfahren aus dem Sumpf einer Demethanisierungskolonne und bei einem Demethanizer-First-Verfahren vom Kopf einer Deethanisierungskolonne oder einem der Deethanisierungskolonne zugeordneten Apparat bereitgestellt werden. Wie erwähnt, kann die Demethanisierung bzw. die Deethanisierung in der hier verwendeten Systematik auch zumindest teilweise als ein Teil des oder der zweiten Vortrennschritte V2 angesehen werden.

Wie bereits erwähnt, können in dem oder den ersten Vortrennschritten V1 und in dem oder den zweiten Vortrennschritten V2 jeweils Stoffströme C2 und C2' abgetrennt werden, die überwiegend oder ausschließlich Kohlenwasserstoffe mit zwei Kohlenstoffatomen umfassen. Dies ist jedoch alternativ oder zusätzlich auch erst in dem oder den ersten Trennschritten möglich. Letzteres ist beispielsweise der Fall, wenn in dem oder den zweiten Trennschritten V2 ein Demethanizer-First-Verfahren eingesetzt wird. Auch in diesem Fall kann eine Zuspeisung des dritten Komponentengemischs C in den Bereich eines sumpfnahen Bodens einer Demethanisierungskolonne erfolgen. Bereits an dieser Stelle werden daher das dritte Komponentengemisch C und das vierte Komponentengemisch D vereinigt. In diesem Fall kann die sich anschließende Deethanisierung systematisch als Teil des oder der ersten Trennschritte angesehen werden, so dass, wie hier in Form eines gestrichelt dargestellten Stoffstroms C2" gezeigt, erst dort ein überwiegend oder ausschließlich Kohlenwasserstoffe mit zwei Kohlenstoffatomen enthaltender Stoffstrom gebildet wird. Die Stoffströme C2' und C2" werden damit vorteilhafterweise alternativ zueinander gebildet, der Stoffstrom C2 kann zusätzlich hierzu gebildet werden.

Figur 2 veranschaulicht einen erfindungsgemäß ausgestalteten Prozess in vereinfachter schematischer Darstellung, die jedoch gegenüber der Darstellung in Figur 1 weitere Details einer spezifischen Ausführungsform zeigt. Baulich identische oder vergleichbare bzw. funktionell identische bzw. vergleichbare Elemente sind dabei mit identischen Bezugszeichen wie in Figur 1 angegeben und werden der Übersichtlichkeit halber nicht wiederholt erläutert.

Wie in Figur 2 veranschaulicht, wird in dem oder den ersten Vortrennschritten V1 das erste Komponentengemisch A zunächst in einem Verdichter 101 verdichtet und anschließend in einem Wärmetauscher 102 abgekühlt. Hierbei wird zumindest ein Teil der in dem ersten Komponentengemisch A enthaltenen Kohlenwasserstoffe mit drei und gegebenenfalls zwei Kohlenstoffatomen auskondensiert.

Zur Phasentrennung kann das entsprechend verdichtete und abgekühlte erste Komponentengemisch A in einen Phasenabscheider 103 überführt werden. Vom Kopf das Phasenabscheiders 103 kann der bereits in Figur 1 dargestellte, mit H2 bezeichnete Stoffstrom abgezogen werden. Aus dem Sumpf des Phasenabscheiders 103 wird das dritte Komponentengemisch C abgezogen, das mittels einer Pumpe 104 weiterbefördert werden kann.

Der oder die zweiten Vortrennschritte V2 umfassen ebenfalls zunächst eine Verdichtung des zweiten Komponentengemischs B unter Verwendung eines Verdichters 105. Der Verdichtung schließt sich eine Trocknung in einem Trockner 106 an. Außerdem kann vor bzw. zwischen der Verdichtung oder Trocknung auch eine Entfernung von Kohlendioxid erfolgen (nicht im Bild gezeigt). Stromab der Trocknung bzw. Entfernung von Kohlendioxid erfolgt eine Kühlung des zweiten Komponentengemischs B unter Verwendung eines Wärmetauschers 107.

Das entsprechend getrocknete und gegebenenfalls von Kohlendioxid befreite zweite Komponentengemisch B wird nun einer Deethanisierung zugeführt, die in Figur 2 veranschaulichte Ausführungsform verwendet also ein Deethanizer-First-Verfahren. Hierbei wird das Komponentengemisch B zunächst in eine Absorberkolonne 108, die mit einem flüssigen Rücklauf gespeist wird, der unter Verwendung eines Kopfgases der eigentlichen Deethanisierungskolonne 109 gebildet wird. Der Deethanisierungskolonne wird ein Sumpfprodukt der Absorberkolonne 108 zugeführt. In der Deethanisierungskolonne 109 bildet sich im Bereich eines sumpfnahen Bodens 109' eine Flüssigkeit, die in der hier verwendeten Systematik das vierte Komponentengemisch D darstellt. Unter dieser Stelle wird das dritte Komponentengemisch C eingespeist. Ein vom Kopf der Deethanisierungskolonne 109, die mit einem Sumpfverdampfer betrieben wird, kann ein Kopfgas abgezogen, in einem insgesamt mit 110 bezeichneten Kopfkondensator verflüssigt und zu einem ersten Teil auf die Absorberkolonne 108 und zu einem anderen Teil auf die Deethanisierungskolonne 109 zurückgeführt werden.

Aus dem Sumpf der Deethanisierungskolonne 109 kann ein überwiegend oder ausschließlich Kohlenwasserstoffe mit drei und mehr Kohlenstoffatomen enthaltender Stoffstrom abgezogen werden, der hier mit C3+ bezeichnet ist, vom Kopf der Absorberkolonne 108 und damit als gasförmige Fraktion, die in der Deethanisierung gebildet wird, kann der bereits in Figur 1 veranschaulichte Stoffstrom C2' abgezogen werden. Ersterer kann dem oder den ersten Trennschritten S1, letzterer dem oder den zweiten Trennschritten S2 zugeführt werden, deren jeweilige Trennungsprodukte P1 bis P4 in Figur 2 nicht gesondert veranschaulicht sind.

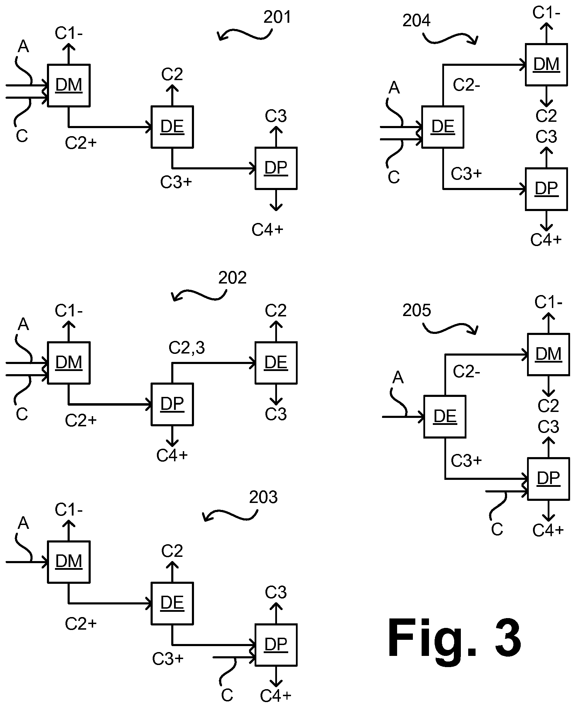

In Figur 3 sind unterschiedliche Ausgestaltungen von erfindungsgemäßen Verfahren stark vereinfacht veranschaulicht. Hierbei sind die unterschiedlichen Einspeisemöglichkeiten für das dritte Komponentengemisch in wahlweise die Demethanisierung (DM), die Deethanisierung (DE) und die Depropanisierung (DP) veranschaulicht. Mit C1- sind dabei überwiegend oder ausschließlich Wasserstoff und Methan enthaltene Komponentengemische, mit C2- überwiegend oder ausschließlich Kohlenwasserstoffe mit zwei Kohlenstoffatomen und leichtere Komponenten enthaltende Komponentengemische, mit C2 überwiegend oder ausschließlich Kohlenwasserstoffe mit zwei Kohlenstoffatomen enthaltende Komponentengemische, mit C3- überwiegend oder ausschließlich Kohlenwasserstoffe mit drei Kohlenstoffatomen und leichtere Komponenten enthaltende Komponentengemische, mit C3 überwiegend oder ausschließlich Kohlenwasserstoffe mit drei Kohlenstoffatomen enthaltende Komponentengemische, mit C2+ überwiegend oder ausschließlich Kohlenwasserstoffe mit zwei und mehr Kohlenstoffatomen enthaltende Komponentengemische, mit C3+ überwiegend oder ausschließlich Kohlenwasserstoffe mit drei und mehr Kohlenstoffatomen enthaltende Komponentengemische, mit C4+ überwiegend oder ausschließlich Kohlenwasserstoffe mit vier und mehr Kohlenstoffatomen enthaltende Komponentengemische und mit C2,3 überwiegend oder ausschließlich Kohlenwasserstoffe mit zwei und drei Kohlenstoffatomen enthaltende Komponentengemische angegeben. Die bereits in den Figuren 1 und 2 mit A bis D bezeichneten Komponentengemische sind auch hier veranschaulicht und entsprechend bezeichnet.

Gemäß Alternative 201 wird ein Demethanizer-First-Verfahren eingesetzt. In den Bereich eines sumpfnahen Trennbodens einer in der Demethanisierung DM verwendeten Demethanisierungskolonne wird dabei das dritte Komponentengemisch C eingespeist. Die übrigen Schritte der Deethanisierung DE und der Depropanisierung DP schließen sich in grundsätzlich bekannter Weise an.

Gemäß Alternative 202 wird ebenfalls ein Demethanizer-First-Verfahren eingesetzt. In den Bereich eines sumpfnahen Trennbodens einer in der Demethanisierung DM verwendeten Demethanisierungskolonne wird dabei auch hier das dritte Komponentengemisch eingespeist. Im Gegensatz zu der Alternative 201 schließen sich jedoch zunächst die Depropanisierung DP und anschließend die Deethanisierung DE, jedoch ebenfalls in grundsätzlich bekannter Weise, an.

Auch gemäß Alternative 203 wird ein Demethanizer-First-Verfahren eingesetzt, wobei die Reihenfolge grundsätzlich jener gemäß Alternative 201 gleicht, jedoch das dritte Komponentengemisch C einer in der Depropanisierung DP verwendeten Depropanisierungskolonne eingespeist wird. Entsprechend könnte das Kopfprodukt dieser Depropanisierung nun auch geringe Mengen an Wasserstoff, Methan und Kohlenwasserstoffen mit zwei Kohlenstoffatomen enthalten, sofern diese aus dem Komponentengemisch C nicht vorher vollständig entfernt wurden. Diese leichten Komponenten könnten dann über einen der nachfolgenden Hydrierung nachgeschalteten Stripper entfernt werden. Das Kopfgas dieses Strippers, das nun das Ethylen sowie das Ethan aus dem ersten Komponentengemisch A enthält, könnte dann direkt dem oder den zweiten Trennschritten S2 oder indirekt über eine erneute Verdichtung in dem oder den ersten Vortrennschritten V1 oder V2 dem oder den zweiten Trennschritten S2 zugeführt werden.

Gemäß den Alternativen 204 und 205 werden jeweils Deethanizer-First-Verfahren eingesetzt, wobei sich der Deethanisierung DE jeweils in grundsätzlich bekannter Weise eine Demethanisierung DM und eine Depropanisierung DP anschließen. Die Alternativen 204 und 205 unterscheiden sich in der jeweiligen Einspeisung des dritten Komponentengemischs C, die in der Alternative 204 in den Bereich eines sumpfnahen Trennbodens einer in der Deethanisierung DE verwendeten Demethanisierungskolonne und in der Alternative 205 in eine in der Depropanisierung DP verwendeten Depropanisierungskolonne eingespeist wird. Für Alternative 205 gilt für das Kopfprodukt dieser Depropanisierung entsprechendes wie für Alternative 203.

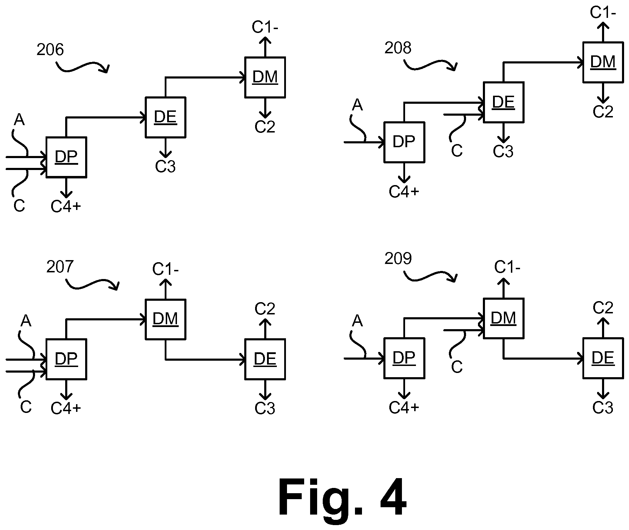

Figur 4 setzt die Darstellung der Figur 3 fort, wobei hier in Form von Alternativen 206 bis 209 jeweils Depropanizer-First-Verfahren veranschaulicht sind. In den nicht erfindungsgemäßen Alternativen 206 und 207 erfolgt die Einspeisung des dritten Komponentengemischs C in eine in der Depropanisierung DP verwendeten Depropanisierungskolonne, in der Alternative 208 in den Bereich eines sumpfnahen Trennbodens einer in der Deethanisierung DE verwendeten Demethanisierungskolonne und in der Alternative 209 in den Bereich eines sumpfnahen Trennbodens einer in der Demethanisierung verwendeten Demethanisierungskolonne. Die Abfolge der übrigen Schritte ergibt sich unmittelbar aus der Zeichnung. Für Alternative 208 und 209 gelangen die geringen Mengen an Kohlenwasserstoffen mit vier Kohlenstoffatomen aus dem Komponentengemisch C schließlich in das überwiegend Propan enthaltene Trennungsprodukt P2. Dies ist nicht weiter kritisch, sofern das Trennprodukt P2 als Recycle zum Dampfspaltverfahren verwendet wird.

Schließlich kann wie bereits erwähnt das Komponentengemisch C auch unabhängig von den oben beschreiben Trennsequenzen stromauf der Verarbeitung von Kohlenwasserstoffen mit drei Kohlenstoffatomen, beispielsweise vor der Hydrierung entsprechender Kohlenwasserstoffe, eingespeist werden (nicht in der Grafik gezeigt). Entsprechend müssten noch die Kohlenwasserstoffe mit zwei und weniger Kohlenstoffatomen, wie für Alternative 203 und 205 beschrieben, dem oder den zweiten Trennschritten S2 zugeführt werden. Außerdem wären geringe Mengen an Kohlenwasserstoffen mit vier und mehr Kohlenstoffatomen im Trennungsprodukt P2 zu tolerieren, wie für Alternativen 208 und 209 beschrieben.

## Patentansprüche

1. Prozess (10) zur Herstellung von Propylen, der umfasst:
- Durchführen eines Verfahrens (1) zur Propandehydrierung unter Erhalt eines ersten Komponentengemischs (A), das zumindest Wasserstoff, Ethan, Ethylen, Propan und Propylen enthält,
- Durchführen eines Dampfspaltverfahrens (2) unter Erhalt eines zweiten Komponentengemischs (B), das zumindest Wasserstoff, Methan, Ethan, Ethylen, Propan und Propylen enthält,
- Bilden eines ersten Trennungsprodukts (P1), das zumindest überwiegend Propylen enthält, unter Verwendung zumindest eines Teils des Propylens des ersten und des zweiten Komponentengemischs (A, B) und unter Einsatz eines oder mehrerer erster Trennschritte (S1),
- Bilden eines zweiten Trennungsprodukts (P2), das zumindest überwiegend Propan enthält, unter Verwendung zumindest eines Teils des Propans des ersten und des zweiten Komponentengemischs (A, B) und unter Einsatz des oder der ersten Trennschritte (S1),
- Bilden eines dritten Trennungsprodukts (P3), das zumindest überwiegend Ethylen enthält, unter Verwendung zumindest eines Teils des Ethylens des ersten und des zweiten Komponentengemischs (A, B) und unter Einsatz eines oder mehrerer zweiter Trennschritte (S2),
- Bilden eines vierten Trennungsprodukts (P4), das zumindest überwiegend Ethan enthält, unter Verwendung zumindest eines Teils des Ethans des ersten und des zweiten Komponentengemischs (A, B) und unter Einsatz des oder der zweiten Trennschritte (S1), und
**dadurch gekennzeichnet,**
- **dass** zumindest ein Teil des ersten Komponentengemischs (A) unter Erhalt eines dritten Komponentengemischs (C) einem oder mehreren ersten Vortrennschritten (V1) unterworfen wird, der oder die eine Druckerhöhung und eine zumindest teilweise Entfernung von Wasserstoff umfassen,
- **dass** zumindest ein Teil des zweiten Komponentengemischs (B) unter Erhalt eines vierten Komponentengemischs (D) einem oder mehreren zweiten Vortrennschritten (V2) unterworfen wird, der oder die eine Druckerhöhung, eine zumindest teilweise Entfernung von Wasserstoff und eine zumindest teilweise Entfernung von Methan umfassen, und
- **dass** zumindest ein Teil des dritten Komponentengemischs (C) gemeinsam mit zumindest einem Teil des vierten Komponentengemischs (D) dem oder den ersten Trennschritten (S1) unterworfen werden.

2. Prozess (10) nach Anspruch 1, bei dem bei der Entfernung von Wasserstoff und Methan in dem oder den zweiten Vortrennschritten (V2) auch Ethan und Ethylen zumindest überwiegend entfernt werden, wobei die Entfernung von Wasserstoff und Methan in dem oder den zweiten Vortrennschritten (V2), bei der auch Ethan und Ethylen zumindest überwiegend entfernt werden, unter Verwendung einer Deethanisierungskolonne (DE) durchgeführt wird.

3. Prozess (10) nach Anspruch 2, bei dem das dritte Komponentengemisch zumindest teilweise in flüssigem Zustand in den Bereich eines sumpfnahen Trennbodens der Deethanisierungskolonne (DE) eingespeist wird.

4. Prozess (10) nach Anspruch 1, bei dem bei der Entfernung von Wasserstoff und Methan in dem oder den zweiten Vortrennschritten (V2) Ethan und Ethylen zumindest überwiegend nicht entfernt werden, wobei die Entfernung von Wasserstoff und Methan in dem oder den zweiten Vortrennschritten (V2), bei der Ethan und Ethylen zumindest überwiegend nicht entfernt werden, unter Verwendung einer Demethanisierungskolonne (DM) durchgeführt wird.

5. Prozess (10) nach Anspruch 4, bei dem das dritte Komponentengemisch zumindest teilweise in flüssigem Zustand in den Bereich eines sumpfnahen Trennbodens der Demethanisierungskolonne (DM) eingespeist wird.

6. Prozess (10) nach Anspruch 4 oder 5, bei dem aus dem Sumpf der Demethanisierungskolonne (DM) eine Sumpfflüssigkeit abgezogen und zumindest teilweise in eine Deethanisierungskolonne (DM) überführt wird.

7. Prozess (10) nach Anspruch 2 oder 6, bei dem aus dem Sumpf der Deethanisierungskolonne (DE) eine Sumpfflüssigkeit abgezogen und zumindest teilweise in eine Depropanisierungskolonne (DP) überführt wird.

8. Prozess (10) nach Anspruch 7, bei dem das dritte Komponentengemisch zumindest teilweise in flüssigem Zustand der Depropanisierungskolonne (DP) eingespeist wird.

9. Prozess (10) nach Anspruch 8, bei dem eine Propan und Propylen enthaltende Fraktion vom Kopf der Depropanisierungskolonne (DP) oder einem der Depropanisierungskolonne (DP) zugeordneten Apparat abgezogen und unter Zugabe von Wasserstoff und unter Erhalt einer hydrierten Fraktion zumindest teilweise einer Hydrierung unterworfen wird, wobei nach der Hydrierung leichter als Propan und Propylen siedende Komponenten aus der hydrierten Fraktion zumindest teilweise ausgetrieben werden.

10. Prozess (10) nach einem der vorstehenden Ansprüche, bei dem in dem oder den ersten Vortrennschritten (V1) des ersten Komponentengemischs dessen Wasserstoffgehalt auf einen Wert von 0 bis 10 mol%, insbesondere 0,1 bis 5 mol%, beispielsweise 0,2 bis 2 mol% abgereichert wird.

11. Prozess (10) nach einem der vorstehenden Ansprüche, bei dem der oder die ersten Vortrennschritte, denen das erste Komponentengemisch unterworfen wird, eine Druckerhöhung auf einen Absolutdruck von 3 bis 40 bar, insbesondere von 10 bis 30 bar, beispielsweise von 12 bis 30 bar, umfassen.

12. Prozess (10) nach Anspruch 11, bei dem in dem oder den ersten Vortrennschritten nach der Druckerhöhung eine zumindest teilweise Kondensation von schwerer als Wasserstoff siedenden Komponenten durchgeführt wird.

13. Anlage zur Herstellung von Propylen mit:
- einer ersten Reaktoreinheit, die zum Durchführen eines Verfahrens (1) zur Propandehydrierung unter Erhalt eines ersten Komponentengemischs (A), das zumindest Wasserstoff, Ethan, Ethylen, Propan und Propylen enthält, bereitgestellt und eingerichtet ist,
- einer zweiten Reaktoreinheit, die zum Durchführen eines Dampfspaltverfahrens (2) unter Erhalt eines zweiten Komponentengemischs (B), das zumindest Wasserstoff, Methan, Ethan, Ethylen, Propan und Propylen enthält, bereitgestellt und eingerichtet ist,
- einer ersten Trenneinheit, die zum Bilden eines ersten Trennungsprodukts (P1), das zumindest überwiegend Propylen enthält, unter Verwendung zumindest eines Teils des Propylens des ersten und des zweiten Komponentengemischs (A, B) und unter Einsatz eines oder mehrerer erster Trennschritte (S1) bereitgestellt und eingerichtet ist,
- wobei die erste Trenneinheit ferner zum Bilden eines zweiten Trennungsprodukts (P2), das zumindest überwiegend Propan enthält, unter Verwendung zumindest eines Teils des Propans des ersten und des zweiten Komponentengemischs (A, B) und unter Einsatz des oder der ersten Trennschritte (S1) bereitgestellt und eingerichtet ist,
- einer zweiten Trenneinheit, die zum Bilden eines dritten Trennungsprodukts (P3), das zumindest überwiegend Ethylen enthält, unter Verwendung zumindest eines Teils des Ethylens des ersten und des zweiten Komponentengemischs (A, B) und unter Einsatz eines oder mehrerer zweiter Trennschritte (S2) bereitgestellt und eingerichtet ist,
- wobei die zweite Trenneinheit ferner zum Bilden eines vierten Trennungsprodukts (P4), das zumindest überwiegend Ethan enthält, unter Verwendung zumindest eines Teils des Ethans des ersten und des zweiten Komponentengemischs (A, B) und unter Einsatz des oder der zweiten Trennschritte (S1) bereitgestellt und eingerichtet ist, und
**gekennzeichnet durch**
- eine erste Vortrenneinheit, die dafür bereitgestellt und eingerichtet ist, zumindest einen Teil des ersten Komponentengemischs (A) unter Erhalt eines dritten Komponentengemischs (C) einem oder mehreren ersten Vortrennschritten (V1) zu unterwerfen, der oder die eine Druckerhöhung und eine zumindest teilweise Entfernung von Wasserstoff umfassen,
- eine zweite Vortrenneinheit, die dafür bereitgestellt und eingerichtet ist, zumindest ein Teil des zweiten Komponentengemischs (B) unter Erhalt eines vierten Komponentengemischs (D) einem oder mehreren zweiten Vortrennschritten (V2) zu unterwerfen, der oder die eine Druckerhöhung, eine zumindest teilweise Entfernung von Wasserstoff und eine zumindest teilweise Entfernung von Methan umfassen, und
- Mittel, die dafür bereitgestellt und eingerichtet sind, zumindest einen Teil des dritten Komponentengemischs (C) gemeinsam mit zumindest einem Teil des vierten Komponentengemischs (D) der ersten Trenneinheit zuzuführen und dem oder den ersten Trennschritten (S1) zu unterwerfen.

14. Verfahren zum Umrüsten einer Anlage, die zur Durchführung eines Dampfspaltverfahrens unter Verwendung einer Anzahl von Anlagenkomponenten eingerichtet ist, wobei der Anlage vor der Umrüstung ein Kohlenwasserstoffe enthaltendes Einsatzgemisch mit einer ersten Zusammensetzung zugeführt wird, **dadurch gekennzeichnet, dass** die Umrüstung umfasst, der Anlage anstelle des Kohlenwasserstoffe enthaltenden Einsatzgemischs mit der ersten Zusammensetzung ein Kohlenwasserstoffe enthaltendes Einsatzgemisch mit einer zweiten, abweichenden Zusammensetzung zuzuführen, und eine oder mehrere der Anlagenkomponenten anstatt für das Dampfspaltverfahren für ein Verfahren zur Propandehydrierung zu nutzen, wobei ein Verfahren nach einem der Ansprüche 1 bis 12 durchgeführt und/oder eine Anlage nach Anspruch 13 bereitgestellt wird..

## Claims

1. Process (10) for producing propylene, comprising:
- carrying out a process (1) for propane dehydrogenation to obtain a first constituent mixture (A) containing at least hydrogen, ethane, ethylene, propane and propylene,
- carrying out a steam cracking process (2) to obtain a second constituent mixture (B) containing at least hydrogen, methane, ethane, ethylene, propane and propylene,
- forming a first separation product (P1) containing at least predominantly propylene using at least a portion of the propylene of the first and of the second constituent mixture (A, B) and using one or more first separation steps (S1),
- forming a second separation product (P2) containing at least predominantly propane using at least a portion of the propane of the first and of the second constituent mixture (A, B) and using the first separation step or steps (S1),
- forming a third separation product (P3) containing at least predominantly ethylene using at least a portion of the ethylene of the first and of the second constituent mixture (A, B) and using one or more second separation steps (S2),
- forming a fourth separation product (P4) containing at least predominantly ethane using at least a portion of the ethane of the first and of the second constituent mixture (A, B) and using the second separation step or steps (S1), and **characterized in**
- **that** at least a portion of the first constituent mixture (A) is subjected to one or more first pre-separation steps (V1) comprising a pressure increase and an at least partial removal of hydrogen to obtain a third constituent mixture (C),
- **that** at least a portion of the second constituent mixture (B) is subjected to one or more second pre-separation steps (V2) comprising a pressure increase, an at least partial removal of hydrogen and an at least partial removal of methane to obtain a fourth constituent mixture (D), and
- **that** at least a portion of the third constituent mixture (C) is subjected to the first separation step or steps (S1) together with at least a portion of the fourth constituent mixture (D).

2. Process (10) according to claim 1, wherein during the removal of hydrogen and methane in the second pre-separation step or steps (V2) ethane and ethylene are also at least predominantly removed, wherein the removal of hydrogen and methane in the second pre-separation step or steps (V2), in which ethane and ethylene are also at least predominantly removed, is carried out using a deethanizer column (DE).

3. Process (10) according to claim 2, wherein the third constituent mixture is at least in part fed in a liquid state into the region of a separating tray in the proximity of the bottom of the deethanizer column (DE).

4. Process (10) according to claim 1, wherein during the removal of hydrogen and methane in the second pre-separation step or steps (V2), ethane and ethylene are at least predominantly not removed, wherein the removal of hydrogen and methane in the second pre-separation step or steps (V2), in which ethane and ethylene are at least predominantly not removed, is carried out using a demethanizer column (DM).

5. Process (10) according to claim 4, wherein the third constituent mixture is at least in part fed in a liquid state into the region of a separating tray in the proximity of the bottom of the demethanizer column (DM).

6. Process (10) according to claim 4 or 5, wherein a bottoms liquid is withdrawn from the bottom of the demethanizer column (DM) and transferred at least in part to a deethanizer column (DM).

7. Process (10) according to claim 2 or 6, wherein a bottoms liquid is withdrawn from the bottom of the deethanizer column (DE) and transferred at least in part to a depropanizer column (DP).

8. Process (10) according to claim 7, wherein the third constituent mixture is at least in part fed in a liquid state to the depropanizer column (DP).

9. Process (10) according to claim 8, wherein a fraction containing propane and propylene is withdrawn from the head of the depropanizer column (DP) or an apparatus associated with the depropanizer column (DP) and, with the addition of hydrogen, subjected at least in part to hydrogenation to obtain a hydrogenated fraction, wherein constituents with a lower boiling point than propane and propylene are at least in part expelled from the hydrogenated fraction after hydrogenation.

10. Process (10) according to any one of the preceding claims, wherein in the first pre-separation step or steps (V1) of the first constituent mixture its hydrogen content is depleted to a value of 0 to 10 mol%, in particular 0.1 to 5 mol%, for example 0.2 to 2 mol%.

11. Process (10) according to any one of the preceding claims, wherein the first pre-separation step or steps to which the first constituent mixture is subjected comprise a pressure increase to an absolute pressure of 3 to 40 bar, in particular of 10 to 30 bar, for example of 12 to 30 bar.

12. Process (10) according to claim 11, wherein in the first pre-separation step or steps after the pressure increase an at least partial condensation of constituents with a higher boiling point than hydrogen is carried out.

13. Plant for producing propylene, comprising:
- a first reactor unit provided and configured to carry out a process (1) for propane dehydrogenation to obtain a first constituent mixture (A) containing at least hydrogen, ethane, ethylene, propane and propylene,
- a second reactor unit provided and configured to carry out a steam cracking process (2) to obtain a second constituent mixture (B) containing at least hydrogen, methane, ethane, ethylene, propane and propylene,
- a first separation unit provided and configured to form a first separation product (P1) containing at least predominantly propylene using at least a portion of the propylene of the first and of the second constituent mixture (A, B) and using one or more first separation steps (S1),
- wherein the first separation unit is further provided and configured to form a second separation product (P2) containing at least predominantly propane using at least a portion of the propane of the first and of the second constituent mixture (A, B) and using the first separation step or steps (S1),
- a second separation unit provided and configured to form a third separation product (P3) containing at least predominantly ethylene using at least a portion of the ethylene of the first and of the second constituent mixture (A, B) and using one or more second separation steps (S2),
- wherein the second separation unit is further provided and configured to form a fourth separation product (P4) containing at least predominantly ethane using at least a portion of the ethane of the first and of the second constituent mixture (A, B) and using the second separation step or steps (S1), and **characterized by**
- a first pre-separation unit provided and configured to subject at least a portion of the first constituent mixture (A) to one or more first pre-separation steps (V1) comprising a pressure increase and an at least partial removal of hydrogen to obtain a third constituent mixture (C),
- a second pre-separation unit provided and configured to subject at least a portion of the second constituent mixture (B) to one or more second pre-separation steps (V2) comprising a pressure increase, an at least partial removal of hydrogen and an at least partial removal of methane to obtain a fourth constituent mixture (D), and
- means provided and configured to feed at least a portion of the third constituent mixture (C) together with at least a portion of the fourth constituent mixture (D) to the first separation unit and to subject it to the first separation step or steps (S1).

14. Method for retrofitting a plant configured to carry out a steam cracking process using a number of plant components, wherein before the retrofitting a hydrocarbon-containing feed mixture with a first composition is fed to the plant, **characterized in that** the retrofitting comprises feeding to the plant a hydrocarbon-containing feed mixture with a second, different composition instead of the hydrocarbon-containing feed mixture with the first composition, and using one or more of the plant components for a process for propane dehydrogenation instead of for the steam cracking process, wherein a process according to any of claims 1 to 12 is carried out and/or a plant according to claim 13 is provided.

## Revendications

1. Procédé (10) de production de propylène comprenant :
- la mise en œuvre d'un procédé (1) de déshydrogénation du propane avec obtention d'un premier mélange de composants (A), lequel contient au moins de l'hydrogène, de l'éthane, de l'éthylène, du propane et du propylène,
- la mise en œuvre d'un procédé de craquage à la vapeur (2) avec obtention un deuxième mélange de composants (B), lequel contient au moins de l'hydrogène, du méthane, de l'éthane, de l'éthylène, du propane et du propylène,
- la formation d'un premier produit de séparation (P1), lequel contient au moins essentiellement du propylène, à l'aide d'au moins une partie du propylène du premier et du deuxième mélange de composants (A, B) et à l'aide d'une ou de plusieurs premières étapes de séparation (S1),
- la formation d'un deuxième produit de séparation (P2), lequel contient au moins essentiellement du propane, à l'aide d'au moins une partie du propane du premier et du deuxième mélange de composants (A, B) et à l'aide de ladite ou desdites premières étapes de séparation (S1),
- la formation d'un troisième produit de séparation (P3), lequel contient au moins essentiellement de l'éthylène, à l'aide d'au moins une partie de l'éthylène du premier et du deuxième mélange de composants (A, B) et à l'aide d'une ou de plusieurs deuxièmes étapes de séparation (S2),
- la formation d'un quatrième produit de séparation (P4), lequel contient au moins essentiellement de l'éthane, à l'aide d'au moins une partie de l'éthane du premier et du deuxième mélange de composants (A, B) et à l'aide de ladite ou desdites deuxièmes étapes de séparation (S1) et
**caractérisé en ce**
- **qu'**au moins une partie du premier mélange de composants (A) est soumise, avec obtention d'un troisième mélange de composants (C), à une ou plusieurs premières étapes de préséparation (V1), laquelle ou lesquelles comprennent une augmentation de pression et une élimination au moins partielle de l'hydrogène,
- **qu'**au moins une partie du deuxième mélange de composants (B) est soumise, avec obtention d'un quatrième mélange de composants (D), à une ou plusieurs deuxièmes étapes de préséparation (V2), laquelle ou lesquelles comprennent une augmentation de pression, une élimination au moins partielle de l'hydrogène et une élimination au moins partielle du méthane et
- **qu'**au moins une partie du troisième mélange de composants (C), ainsi qu'au moins une partie du quatrième mélange de composants (D) sont soumises conjointement à ladite ou auxdites premières étapes de séparation (S1).

2. Procédé (10) selon la revendication 1, selon lequel, lors de l'élimination de l'hydrogène et du méthane dans ladite ou lesdites deuxièmes étapes de préséparation (V2), l'éthane et l'éthylène sont également au moins essentiellement éliminés, l'élimination de l'hydrogène et du méthane dans ladite ou lesdites deuxièmes étapes de préséparation (V2), selon laquelle l'éthane et l'éthylène sont également au moins essentiellement éliminés, étant mise en œuvre à l'aide d'une colonne de déséthanisation (DE).

3. Procédé (10) selon la revendication 2, selon lequel le troisième mélange de composants est injecté au moins partiellement à l'état liquide dans la zone d'un plateau de séparation proche du fond de la colonne de déséthanisation (DE).

4. Procédé (10) selon la revendication 1, selon lequel, lors de l'élimination de l'hydrogène et du méthane dans ladite ou lesdites deuxièmes étapes de préséparation (V2), l'éthane et l'éthylène ne sont pas au moins essentiellement éliminés, l'élimination de l'hydrogène et du méthane dans ladite ou lesdites deuxièmes étapes de préséparation (V2), selon laquelle ou lesquelles l'éthane et l'éthylène ne sont pas au moins essentiellement éliminés, étant mise en œuvre à l'aide d'une colonne de déméthanisation (DM).

5. Procédé (10) selon la revendication 4, selon lequel le troisième mélange de composants est injecté au moins partiellement à l'état liquide dans la zone d'un plateau de séparation à proximité du fond de la colonne de déméthanisation (DM).

6. Procédé (10) selon la revendication 4 ou 5, selon lequel un liquide de fond est soutiré du bas de la colonne de déméthanisation (DM) et au moins partiellement transféré dans une colonne de déséthanisation (DM).

7. Procédé (10) selon la revendication 2 ou 6, selon lequel un liquide de fond est soutiré du bas de la colonne de déséthanisation (DE) et au moins partiellement transféré dans une colonne de dépropanisation (DP).

8. Procédé (10) selon la revendication 7, selon lequel le troisième mélange de composants est injecté au moins partiellement à l'état liquide dans la colonne de dépropanisation (DP).

9. Procédé (10) selon la revendication 8, selon lequel une fraction contenant du propane et du propylène est soutirée de la tête de la colonne de dépropanisation (DP) ou d'un appareil affecté à la colonne de dépropanisation (DP) et est, avec addition d'hydrogène et avec obtention une fraction hydrogénée, au moins partiellement soumise à une hydrogénation, les composants à point d'ébullition inférieur à celui du propane et du propylène étant au moins partiellement éliminés de la fraction hydrogénée après l'hydrogénation.

10. Procédé (10) selon l'une quelconque des revendications précédentes, selon lequel, dans ladite ou lesdites premières étapes de préséparation (V1) du premier mélange de composants, sa teneur en hydrogène est appauvrie à une valeur de 0 à 10 % en mole, en particulier de 0,1 à 5 % en mole, par exemple de 0,2 à 2 % en mole.

11. Procédé (10) selon l'une quelconque des revendications précédentes, selon lequel ladite ou lesdites premières étapes de préséparation, à laquelle ou auxquelles le premier mélange de composants est soumis, comprennent l'augmentation de pression jusqu'à une pression absolue de 3 à 40 bars, en particulier de 10 à 30 bars, par exemple de 12 à 30 bars.

12. Procédé (10) selon la revendication 11, selon lequel, dans ladite ou lesdites premières étapes de préséparation après l'augmentation de pression, une condensation au moins partielle des composants à point d'ébullition supérieur à celui de l'hydrogène est mise en œuvre.

13. Installation de production de propylène comprenant :
- une première unité de réacteur, laquelle est utilisée et conçue pour mettre en œuvre un procédé (1) de déshydrogénation du propane avec obtention d'un premier mélange de composants (A), lequel contient au moins de l'hydrogène, de l'éthane, de l'éthylène, du propane et du propylène,
- une deuxième unité de réacteur, laquelle est utilisée et conçue pour mettre en œuvre un procédé de craquage à la vapeur (2) avec obtention d'un deuxième mélange de composants (B), lequel contient au moins de l'hydrogène, du méthane, de l'éthane, de l'éthylène, du propane et du propylène,
- une première unité de séparation, laquelle est utilisée et conçue pour former un premier produit de séparation (P1), lequel contient au moins essentiellement du propylène, à l'aide d'au moins une partie du propylène du premier et du deuxième mélange de composants (A, B) et à l'aide d'une ou de plusieurs premières étapes de séparation (S1),
- la première unité de séparation étant en outre utilisée et conçue pour former un deuxième produit de séparation (P2), lequel contient au moins essentiellement du propane, à l'aide d'au moins une partie du propane du premier et du deuxième mélange de composants (A, B) et à l'aide de ladite ou desdites premières étapes de séparation (S1),
- une deuxième unité de séparation, laquelle est utilisée et conçue pour former un troisième produit de séparation (P3), lequel contient au moins essentiellement de l'éthylène, à l'aide d'au moins une partie de l'éthylène du premier et du deuxième mélange de composants (A, B) et à l'aide d'un ou de plusieurs deuxièmes étapes de séparation (S2),
- la deuxième unité de séparation étant en outre utilisée et conçue pour former un quatrième produit de séparation (P4), lequel contient au moins essentiellement de l'éthane, à l'aide d'au moins une partie de l'éthane du premier et du deuxième mélange de composants (A, B) et à l'aide de ladite ou desdites étapes de séparation (S1) et
**caractérisé par**
- une première unité de préséparation, laquelle est utilisée et conçue pour soumettre au moins une partie du premier mélange de composants (A), avec obtention d'un troisième mélange de composants (C), à une ou plusieurs premières étapes de préséparation (V1), laquelle ou lesquelles comprennent une augmentation de pression et une élimination au moins partielle de l'hydrogène,
- une deuxième unité de préséparation, laquelle est utilisée et conçue pour soumettre au moins une partie du deuxième mélange de composants (B), avec obtention d'un quatrième mélange de composants (D), à une ou plusieurs deuxièmes étapes de préséparation (V2), laquelle ou lesquelles comprennent une augmentation de pression, une élimination au moins partielle de l'hydrogène et une élimination au moins partielle du méthane et
- des moyens, lesquels sont utilisés et conçus pour alimenter au moins une partie du troisième mélange de composants (C) ainsi qu'au moins une partie du quatrième mélange de composants (D) conjointement à la première unité de séparation et pour les soumettre à ladite ou auxdites premières étapes de séparation (S1).

14. Procédé de modernisation d'une installation, laquelle est conçue pour mettre en œuvre un procédé de craquage à la vapeur à l'aide d'un certain nombre de composants d'installation, l'installation étant alimentée, avant la modernisation, en un mélange de départ contenant des hydrocarbures présentant une première composition, **caractérisé en ce que** la modernisation comprend l'alimentation de l'installation en, au lieu du mélange de départ contenant des hydrocarbures présentant la première composition, un mélange de départ contenant des hydrocarbures présentant une deuxième composition différente et l'utilisation d'un ou de plusieurs des composants d'installation pour, au lieu du procédé de craquage à la vapeur, un procédé de déshydrogénation du propane, un procédé selon l'une quelconque des revendications 1 à 12 étant mis en œuvre et/ou une installation selon la revendication 13 étant utilisée.
